# EUROPEAN PATENT SPECIFICATION

(11) **EP 3 264 906 B1**
(45) Date of publication and mention of the grant of the patent: **06.05.2020**
(21) Application number: 16708170.2
(22) Date of filing: 07.03.2016
(51) Int. Cl.: A23C 9/156, C11D 3/50, C12P 19/44, C12N 9/10

(54) **GLYCOSYLTRANSFERASE, TRANSGENIC PLANT AND METHOD FOR FORMING AROMA GLYCOSIDES**
GLYKOSYLTRANSFERASE, TRANSGENE PFLANZE UND VERFAHREN ZUR HESTELLUNG VON AROMATISCHEN GLYKOSIDEN
TRANSFERASE DE GLYCOSYLE, PLANTE TRANSGENIQUE ET PROCÉDÉ POUR LA PRÉPARATION DES GLYCOSIDES AROMATIQUES

(30) Priority: 06.03.2015 EP 15158057; 20.03.2015 EP 15160115; 20.04.2015 EP 15164326
(43) Date of publication of application: 10.01.2018
(73) Proprietor: Technische Universität München, 80333 München (DE)
(72) Inventor: SCHWAB, Wilfried, 97828 Marktheidenfeld (DE); HOFFMANN, Thomas, 85244 Biberbach (DE); HUANG, Fong-Chin, 85354 Freising (DE); FISCHER, Thilo, 85737 Ismaning (DE); HINZ, Detlef Erich, 85737 Ismaning (DE); ADAMSKI, Heimo, 85465 Langenpreising (DE)
(74) Representative: Isarpatent
(86) International application number: PCT/EP2016/054760
(87) International publication number: WO 2016/142329

(56) References cited:
- WO-A1-2004/111254
- WO-A1-2004/111254
- WO-A1-2006/087370
- DATABASE PROTEIN [Online] 4 March 2015 (2015-03-04), "PREDICTED: anthocyanidin 3-O-glucosyltransferase 2-like [Fragaria vesca subsp. vesca]", XP002786020, retrieved from NCBI Database accession no. XP_004294260
- DATABASE UniProt [Online] 10 August 2010 (2010-08-10), "RecName: Full=Glycosyltransferase {ECO:0000256|RuleBase:RU362057}; EC=2.4.1.- {ECO:0000256|RuleBase:RU362057};", XP002786021, retrieved from EBI accession no. UNIPROT:D7UCM3 Database accession no. D7UCM3
- YAR-KHING YAUK ET AL: "Manipulation of flavour and aroma compound sequestration and release using a glycosyltransferase with specificity for terpene alcohols", THE PLANT JOURNAL, vol. 80, no. 2, 8 September 2014 (2014-09-08), pages 317-330, XP055247654, GB ISSN: 0960-7412, DOI: 10.1111/tpj.12634
- KOEDUKA TAKAO ET AL: "Enhancement of production of eugenol and its glycosides in transgenic aspen plantsviagenetic engineering", BIOCHEMICAL AND BIOPHYSICAL RESEARCH COMMUNICATIONS, ACADEMIC PRESS INC. ORLANDO, FL, US, vol. 436, no. 1, 23 May 2013 (2013-05-23), pages 73-78, XP028570477, ISSN: 0006-291X, DOI: 10.1016/J.BBRC.2013.05.060
- GOSCH C ET AL: "Cloning and heterologous expression of glycosyltransferases from Malus x domestica and Pyrus communis, which convert phloretin to phloretin 2@?-O-glucoside (phloridzin)", PLANT SCIENCE, ELSEVIER IRELAND LTD, IE, vol. 178, no. 3, 1 March 2010 (2010-03-01) , pages 299-306, XP026932497, ISSN: 0168-9452, DOI: 10.1016/J.PLANTSCI.2009.12.009 [retrieved on 2010-01-08] & DATABASE EMBL [Online] 19 February 2010 (2010-02-19), "Pyrus communis clone UGT71K2 glycosyltransferase mRNA, complete cds.", retrieved from EBI accession no. EM_STD:FJ854495 Database accession no. FJ854495
- Y. M. TIKUNOV ET AL: "NON-SMOKY GLYCOSYLTRANSFERASE1 Prevents the Release of Smoky Aroma from Tomato Fruit", THE PLANT CELL, vol. 25, no. 8, 1 August 2013 (2013-08-01) , pages 3067-3078, XP055247660, US ISSN: 1040-4651, DOI: 10.1105/tpc.113.114231
- THOMAS LOUVEAU ET AL: "Predicting the substrate specificity of a glycosyltransferase implicated in the production of phenolic volatiles in tomato fruit", FEBS JOURNAL, vol. 278, no. 2, 17 January 2011 (2011-01-17), pages 390-400, XP055247672, GB ISSN: 1742-464X, DOI: 10.1111/j.1742-4658.2010.07962.x
- JUAN JOSÉ RODRÍGUEZ-BENCOMO ET AL: "Application of glycosidic aroma precursors to enhance the aroma and sensory profile of dealcoholised wines", FOOD RESEARCH INTERNATIONAL., vol. 51, no. 2, 1 May 2013 (2013-05-01), pages 450-457, XP055247707, GB ISSN: 0963-9969, DOI: 10.1016/j.foodres.2012.12.057
- DATABASE Refseq [Online] NCBI; 4 March 2015 (2015-03-04), "PREDICTED: anthocyanidin 3-O-glucosyltransferase 2-like [Fragaria vesca subsp. vesca]", XP002786020, Database accession no. XP_004294260.1
- DATABASE UniProt [Online] 10 August 2010 (2010-08-10), "RecName: Full=Glycosyltransferase {ECO:0000256|RuleBase:RU362057}; EC=2.4.1.- {ECO:0000256|RuleBase:RU362057};", XP002786021, retrieved from EBI accession no. UNIPROT:D7UCM3 Database accession no. D7UCM3
- YAR-KHING YAUK ET AL: "Manipulation of flavour and aroma compound sequestration and release using a glycosyltransferase with specificity for terpene alcohols", THE PLANT JOURNAL, vol. 80, no. 2, 8 September 2014 (2014-09-08), pages 317-330, XP055247654, GB ISSN: 0960-7412, DOI: 10.1111/tpj.12634

### Sequence protocol

### SEQUENCE LISTING

<110> Technische Universität München
<120> Glycosylated aroma- and fragrance precursors and fragrance products that can be activated
<130> P38396-WO
<150> EP15158057.8
   <151> 2015-03-06
<150> EP15164326.9
   <151> 2015-04-20
<150> EP15160115.0
   <151> 2015-03-20
<160> 6
<170> PatentIn version 3.5
<210> 1
   <211> 1437
   <212> DNA
   <213> artificial
<220>
   <223> Glycosyl transferase FaGTs1-5 coding sequence
<400> 1
   atgaagaaag cagagctagt gttcatccct gcaccaggag ccggccacct tgtgtcagcc      60
   ttgcaattcg gaaagcgtct gcttcagcga gatgatcgaa tttcaatcac agttcttgcc     120
   atcaaatcag cagccccttc atctttaggt tcatacacag aagcccttgt agcttccgag     180
   tcccgtctcc aactcattga cgtccctcaa gctgagctcc ctccattaga gtttgcaaag     240
   tcaccagcta agtttttcat tctaaacatt gagaaccatg ttcctaatgt cagagaggcc     300
   ctcactaact atgtctcatc taagcaagac tcggttccaa ttgttggagt ggttcttgat     360
   ttcttctgtg tctccatgat tgatgtggtc aatgaattca atctcccttc ttatctattc     420
   atgacaagca atgcagggta tctttctttc aagttccact ttccggcgca ggatagccgg     480
   accggtcggc cgcctaaaga ctccgatcct gattggttag tccccggtat tgtcccccct     540
   gttcctacca aagttttgcc tgtgtcttta actgatggta gctactataa ttatcttggg     600
   gttgcttcga gatttagaga ggccaaaggt atcatagcaa atacatgtgt tgagctagag     660
   acacatgcgt tcaactcttt tgctgaagat caaactacgc ctccggtgta cccagttgga     720
   ccggtgcttg atctcaacga tggtcaggct cggtccaatt taaaccaggc gcagcgtgac     780
   aagatcatca gctggcttga tgatcagcct gaagagtctg ttgtgttctt atgctttgga     840
   agcatgggga gctttactga agcacaagtg aaggagatag ctctgggcct tgagcagagt     900
   gggcagaggt tcttgtggtc tttgcgtttg acaccaccaa aggggagtaa aagtttgagt     960
   cctgtggact gctcgaacct tgaggaagta ttgccggatg gattcttgga gaggactaga      1020
   gaaaagggac tgatatgcgg gtgggcgccg caggtggacg tcttgtcaca caaggcaacc      1080
   ggaggctttg tgtcgcattg tgggtggaac tcgatcttgg agagcttgtg gcatggcgtg      1140
   ccgattgtga catggcctat gtatgctgag caacagctga atgcgtttcg attggtgaag      1200
   gagatggggt tgggattgga gatgaggttg gattacaagc gaggtggtga tgaggttgtg      1260
   aaggcagatg agattgggaa ggctgtagct agtgtgatgg agaacagtga ggtgaggaag      1320
   aaagtgaaag aaattggggt ggtgtgcaga aaagctgtag aggatggagg gtcttcttct      1380
   gtttcacttg gaaggttcat tgaagatgtg atgaggaatc attttggttc tgagtaa         1437
<210> 2
   <211> 1437
   <212> DNA
   <213> artificial
<220>
   <223> Glycosyl transferase FaGTs1-13 coding sequence
<400> 2
   atgaagaaag cagagctagt gttcatccct gcaccaggag ccggccacct tgtgtcagcc 60
   ttgcaattcg gaaagcgtct gcttcagcga gatgatcgaa tttcaatcac agttcttgcc 120
   atcaaatcag cagccccttc atctttaggt tcatacacag aagcccttgt agcttccgag 180
   tcccgtctcc aactcatcga cgtccctcaa gctgagctcc ctccattaga gtttgcaaag 240
   tcaccagcta agtttttcat tctaaacatt gagaaccatg ttcctaatgt cagagaggcc 300
   ctcactaact atgtctcatc taagcaagac tcggttccaa ttgttggagt ggttcttgat 360
   ttcttctgtg tctccatgat tgatgtggtc aatgaattca atctcccttc ttatctattc 420
   atgacaagca atgcagggta tctttctttc aagttccact ttccggcgca ggatagccgg 480
   accggtcggc cgcctaaaga ctccgatcct gattggttag tccccggtat tgtcccccct 540
   gttcctacca aagttttgcc tgtgtcttta actgatggta gctactataa ttatcttggg 600
   gttgcttcga gatttagaga ggccaaaggt atcatagcaa atacatgtgt tgagctagag 660
   acatatgcgt tcaactcttt tgctgaagat caaactacgc ctccggtgta cccagttgga 720
   ccggtgcttg atctcaacga tggtcaggct cggtccaatt taaaccaggc gcagcgtgac 780
   aagatcatta gctggcttga tgatcagcct gaagagtctg ttgtgttctt atgctttgga 840
   agcatgggga gctttactga agcacaagtg aaggagatag ctctgggcct tgagcagagt 900
   gggcagaggt tcttgtggtc tttgcgtttg acaccaccaa aggggagtaa aagtttgagt 960
   cctgtggaat gctcgaacct tgaggaagta ttgccggatg gattcttgga gaggactaga 1020
   gaaaagggac tgatatgcgg gtgggcgccg caggtggacg tcttgtcaca caaggcaacc 1080
   ggaggctttg tgtcgcattg tgggtggaac tcgatcttgg agagcttgtg gcatggcgtg 1140
   ccgattgtga catggcctat gtatgctgag caacagctga atgcgtttcg attggtgaag 1200
   gagatggggt tgggattgga gatgaggttg gattacaagc gaggtggtga tgaggttgtg 1260
   aaggcagatg agattgggaa ggctgtagct agtgtgatgg agaacagtga ggtgaggaag 1320
   aaagtgaaag aaattggggt ggtgtgcaga aaagctgtag aggatggagg gtcttcttct 1380
   gtttcacttg gaaggttcat tgaagatgtg atgaggaatc attttggttc tgagtaa 1437
<210> 3
   <211> 1410
   <212> DNA
   <213> artificial
<220>
   <223> Glycosyl transferase VvdGT13 coding sequence
<400> 3
   atggctgaaa aacctcccca catagccatt ctcccgaccc ctggcatggg tcacctcatc      60
   cctctcattg aactagccaa acgcctcgtc acccaccatg gattcacagt cactttcatc     120
   atccccaacg ataattcttc tctcaaagcc cagaaggccg tgctccagag cctccctccc     180
   agcatagact ccatttttct tcctccggtt agttttgatg acttgccggc ggagacgaag     240
   atcgagacta tgatttctct cactgtggtt cggtcacttt cccatctccg gagctcgctg     300
   gagttgctgg tttcgaagac ccgagtggcg gcgctcgtcg tcgatctctt cggaaccgac     360
   gcgttcgacg tcgcggcgga gttcggggtg gcgccgtaca ttttcttccc gtcgacggcg     420
   atggctctct cgctgtttct gttccttccg aagctggatg agatggtggc gtgcgaattt     480
   cgggacatga atgaaccggt ggcgattcct ggttgcgtgc cggtccacgg ctcgcagctg     540
   ctcgacccgg ttcaggatag gaggaacgac gcttacaagt gggttctaca ccacaccaag     600
   agatatcgac tggctgaagg gataatggtg aatagcttca tggagttgga gcctggaccg     660
   ctcaaggctc tgcagacacc cgaaccgggt aaaccgccgg tctacccggt cggaccgctg     720
   ataaagaggg agtcggagat gggatccggt gagaacgagt gtttgaagtg gctggacgac     780
   cagccacttg gctccgtcct attcgtcgct ttcgggagcg gtgggaccct ccccagtgag     840
   caattagatg agctggcctt gggcttagag atgagtgagc aaagattttt gtgggtagtg     900
   aggagcccta gtcgtgtggc tgattcctca ttcttcagtg tccattctca aaatgaccct     960
   ttctctttct tacctcaagg gtttgtagat agaaccaaag ggcggggcct cttggtgtca    1020
   tcctgggcac cacaggctca aattattagc cacgcatcca ccggagggtt cttgtctcat    1080
   tgcgggtgga actccactct cgagagcgtt gcctgcggtg tccccatgat cgcttggccg    1140
   ctctacgccg agcaaaaaat gaacgcaata acattaaccg acgacttaaa agtggcattg    1200
   aggccaaaag ttaacgaaaa tggtctaatc gatcgtaatg aaattgctcg aattgttaag    1260
   ggtctgatgg aaggggaaga aggaaagggt gtacgcagtc gaatgaagga ccttaaggac    1320
   gcctccgcaa aagttttgag ccatgatggg tcttctacaa aggcactctc aaccgtggct    1380
   caaaaatgga aggctcacaa gaattattag                                    1410
<210> 4
   <211> 478
   <212> PRT
   <213> artificial
<220>
   <223> Glycosyl transferase FaGTs1-5
<400> 4
   Met Lys Lys Ala Glu Leu Val Phe Ile Pro Ala Pro Gly Ala Gly His
   1               5                   10                  15
   Leu Val Ser Ala Leu Gln Phe Gly Lys Arg Leu Leu Gln Arg Asp Asp
               20                  25                  30
   Arg Ile Ser Ile Thr Val Leu Ala Ile Lys Ser Ala Ala Pro Ser Ser
           35                  40                  45
   Leu Gly Ser Tyr Thr Glu Ala Leu Val Ala Ser Glu Ser Arg Leu Gln
       50                  55                  60
   Leu Ile Asp Val Pro Gln Ala Glu Leu Pro Pro Leu Glu Phe Ala Lys
   65                  70                  75                  80
   Ser Pro Ala Lys Phe Phe Ile Leu Asn Ile Glu Asn His Val Pro Asn
                   85                  90                  95
   Val Arg Glu Ala Leu Thr Asn Tyr Val Ser Ser Lys Gln Asp Ser Val
               100                 105                 110
   Pro Ile Val Gly Val Val Leu Asp Phe Phe Cys Val Ser Met Ile Asp
           115                 120                 125
   Val Val Asn Glu Phe Asn Leu Pro Ser Tyr Leu Phe Met Thr Ser Asn
       130                 135                 140
   Ala Gly Tyr Leu Ser Phe Lys Phe His Phe Pro Ala Gln Asp Ser Arg
   145                 150                 155                 160
   Thr Gly Arg Pro Pro Lys Asp Ser Asp Pro Asp Trp Leu Val Pro Gly
                   165                 170                 175
   Ile Val Pro Pro Val Pro Thr Lys Val Leu Pro Val Ser Leu Thr Asp
               180                 185                 190
   Gly Ser Tyr Tyr Asn Tyr Leu Gly Val Ala Ser Arg Phe Arg Glu Ala
           195                 200                 205
   Lys Gly Ile Ile Ala Asn Thr Cys Val Glu Leu Glu Thr His Ala Phe
       210                 215                 220
   Asn Ser Phe Ala Glu Asp Gln Thr Thr Pro Pro Val Tyr Pro Val Gly
   225                 230                 235                 240
   Pro Val Leu Asp Leu Asn Asp Gly Gln Ala Arg Ser Asn Leu Asn Gln
                   245                 250                 255
   Ala Gln Arg Asp Lys Ile Ile Ser Trp Leu Asp Asp Gln Pro Glu Glu
               260                 265                 270
   Ser Val Val Phe Leu Cys Phe Gly Ser Met Gly Ser Phe Thr Glu Ala
           275                 280                 285
   Gln Val Lys Glu Ile Ala Leu Gly Leu Glu Gln Ser Gly Gln Arg Phe
       290                 295                 300
   Leu Trp Ser Leu Arg Leu Thr Pro Pro Lys Gly Ser Lys Ser Leu Ser
   305                 310                 315                 320
   Pro Val Asp Cys Ser Asn Leu Glu Glu Val Leu Pro Asp Gly Phe Leu
                   325                 330                 335
   Glu Arg Thr Arg Glu Lys Gly Leu Ile Cys Gly Trp Ala Pro Gln Val
               340                 345                 350
   Asp Val Leu Ser His Lys Ala Thr Gly Gly Phe Val Ser His Cys Gly
           355                 360                 365
   Trp Asn Ser Ile Leu Glu Ser Leu Trp His Gly Val Pro Ile Val Thr
       370                 375                 380
   Trp Pro Met Tyr Ala Glu Gln Gln Leu Asn Ala Phe Arg Leu Val Lys
   385                 390                 395                 400
   Glu Met Gly Leu Gly Leu Glu Met Arg Leu Asp Tyr Lys Arg Gly Gly
                   405                 410                 415
   Asp Glu Val Val Lys Ala Asp Glu Ile Gly Lys Ala Val Ala Ser Val
               420                 425                 430
   Met Glu Asn Ser Glu Val Arg Lys Lys Val Lys Glu Ile Gly Val Val
   435 440 445
   Cys Arg Lys Ala Val Glu Asp Gly Gly Ser Ser Ser Val Ser Leu Gly
   450 455 460
   Arg Phe Ile Glu Asp Val Met Arg Asn His Phe Gly Ser Glu
   465 470 475
<210> 5
   <211> 478
   <212> PRT
   <213> artificial
<220>
   <223> Glycosyl transferase FaGTs1-13
<400> 5
   Met Lys Lys Ala Glu Leu Val Phe Ile Pro Ala Pro Gly Ala Gly His
   1               5                   10                  15
   Leu Val Ser Ala Leu Gln Phe Gly Lys Arg Leu Leu Gln Arg Asp Asp
               20                  25                  30
   Arg Ile Ser Ile Thr Val Leu Ala Ile Lys Ser Ala Ala Pro Ser Ser
           35                  40                  45
   Leu Gly Ser Tyr Thr Glu Ala Leu Val Ala Ser Glu Ser Arg Leu Gln
       50                  55                  60
   Leu Ile Asp Val Pro Gln Ala Glu Leu Pro Pro Leu Glu Phe Ala Lys
   65                  70                  75                  80
   Ser Pro Ala Lys Phe Phe Ile Leu Asn Ile Glu Asn His Val Pro Asn
                   85                  90                  95
   Val Arg Glu Ala Leu Thr Asn Tyr Val Ser Ser Lys Gln Asp Ser Val
               100                 105                 110
   Pro Ile Val Gly Val Val Leu Asp Phe Phe Cys Val Ser Met Ile Asp
           115                 120                 125
   Val Val Asn Glu Phe Asn Leu Pro Ser Tyr Leu Phe Met Thr Ser Asn
       130                 135                 140
   Ala Gly Tyr Leu Ser Phe Lys Phe His Phe Pro Ala Gln Asp Ser Arg
   145                 150                 155                 160
   Thr Gly Arg Pro Pro Lys Asp Ser Asp Pro Asp Trp Leu Val Pro Gly
                   165                 170                 175
   Ile Val Pro Pro Val Pro Thr Lys Val Leu Pro Val Ser Leu Thr Asp
               180                 185                 190
   Gly Ser Tyr Tyr Asn Tyr Leu Gly Val Ala Ser Arg Phe Arg Glu Ala
           195                 200                 205
   Lys Gly Ile Ile Ala Asn Thr Cys Val Glu Leu Glu Thr Tyr Ala Phe
       210                 215                 220
   Asn Ser Phe Ala Glu Asp Gln Thr Thr Pro Pro Val Tyr Pro Val Gly
   225                 230                 235                 240
   Pro Val Leu Asp Leu Asn Asp Gly Gln Ala Arg Ser Asn Leu Asn Gln
                   245                 250                 255
   Ala Gln Arg Asp Lys Ile Ile Ser Trp Leu Asp Asp Gln Pro Glu Glu
               260                 265                 270
   Ser Val Val Phe Leu Cys Phe Gly Ser Met Gly Ser Phe Thr Glu Ala
           275                 280                 285
   Gln Val Lys Glu Ile Ala Leu Gly Leu Glu Gln Ser Gly Gln Arg Phe
       290                 295                 300
   Leu Trp Ser Leu Arg Leu Thr Pro Pro Lys Gly Ser Lys Ser Leu Ser
   305                 310                 315                 320
   Pro Val Glu Cys Ser Asn Leu Glu Glu Val Leu Pro Asp Gly Phe Leu
                   325                 330                 335
   Glu Arg Thr Arg Glu Lys Gly Leu Ile Cys Gly Trp Ala Pro Gln Val
               340                 345                 350
   Asp Val Leu Ser His Lys Ala Thr Gly Gly Phe Val Ser His Cys Gly
           355                 360                 365
   Trp Asn Ser Ile Leu Glu Ser Leu Trp His Gly Val Pro Ile Val Thr
       370                 375                 380
   Trp Pro Met Tyr Ala Glu Gln Gln Leu Asn Ala Phe Arg Leu Val Lys
   385                 390                 395                 400
   Glu Met Gly Leu Gly Leu Glu Met Arg Leu Asp Tyr Lys Arg Gly Gly
                   405                 410                 415
   Asp Glu Val Val Lys Ala Asp Glu Ile Gly Lys Ala Val Ala Ser Val
   420 425 430
   Met Glu Asn Ser Glu Val Arg Lys Lys Val Lys Glu Ile Gly Val Val
   435 440 445
   Cys Arg Lys Ala Val Glu Asp Gly Gly Ser Ser Ser Val Ser Leu Gly
   450 455 460
   Arg Phe Ile Glu Asp Val Met Arg Asn His Phe Gly Ser Glu
   465 470 475
<210> 6
   <211> 469
   <212> PRT
   <213> artificial
<220>
   <223> Glyosyl transferase VvdGT13
<400> 6
   Met Ala Glu Lys Pro Pro His Ile Ala Ile Leu Pro Thr Pro Gly Met
   1               5                   10                  15
   Gly His Leu Ile Pro Leu Ile Glu Leu Ala Lys Arg Leu Val Thr His
               20                  25                  30
   His Gly Phe Thr Val Thr Phe Ile Ile Pro Asn Asp Asn Ser Ser Leu
           35                  40                  45
   Lys Ala Gln Lys Ala Val Leu Gln Ser Leu Pro Pro Ser Ile Asp Ser
       50                  55                  60
   Ile Phe Leu Pro Pro Val Ser Phe Asp Asp Leu Pro Ala Glu Thr Lys
   65                  70                  75                  80
   Ile Glu Thr Met Ile Ser Leu Thr Val Val Arg Ser Leu Ser His Leu
                   85                  90                  95
   Arg Ser Ser Leu Glu Leu Leu Val Ser Lys Thr Arg Val Ala Ala Leu
               100                 105                 110
   Val Val Asp Leu Phe Gly Thr Asp Ala Phe Asp Val Ala Ala Glu Phe
           115                 120                 125
   Gly Val Ala Pro Tyr Ile Phe Phe Pro Ser Thr Ala Met Ala Leu Ser
       130                 135                 140
   Leu Phe Leu Phe Leu Pro Lys Leu Asp Glu Met Val Ala Cys Glu Phe
   145                 150                 155                 160
   Arg Asp Met Asn Glu Pro Val Ala Ile Pro Gly Cys Val Pro Val His
                   165                 170                 175
   Gly Ser Gln Leu Leu Asp Pro Val Gln Asp Arg Arg Asn Asp Ala Tyr
               180                 185                 190
   Lys Trp Val Leu His His Thr Lys Arg Tyr Arg Leu Ala Glu Gly Ile
           195                 200                 205
   Met Val Asn Ser Phe Met Glu Leu Glu Pro Gly Pro Leu Lys Ala Leu
       210                 215                 220
   Gln Thr Pro Glu Pro Gly Lys Pro Pro Val Tyr Pro Val Gly Pro Leu
   225                 230                 235                 240
   Ile Lys Arg Glu Ser Glu Met Gly Ser Gly Glu Asn Glu Cys Leu Lys
                   245                 250                 255
   Trp Leu Asp Asp Gln Pro Leu Gly Ser Val Leu Phe Val Ala Phe Gly
               260                 265                 270
   Ser Gly Gly Thr Leu Pro Ser Glu Gln Leu Asp Glu Leu Ala Leu Gly
           275                 280                 285
   Leu Glu Met Ser Glu Gln Arg Phe Leu Trp Val Val Arg Ser Pro Ser
       290                 295                 300
   Arg Val Ala Asp Ser Ser Phe Phe Ser Val His Ser Gln Asn Asp Pro
   305                 310                 315                 320
   Phe Ser Phe Leu Pro Gln Gly Phe Val Asp Arg Thr Lys Gly Arg Gly
                   325                 330                 335
   Leu Leu Val Ser Ser Trp Ala Pro Gln Ala Gln Ile Ile Ser His Ala
               340                 345                 350
   Ser Thr Gly Gly Phe Leu Ser His Cys Gly Trp Asn Ser Thr Leu Glu
           355                 360                 365
   Ser Val Ala Cys Gly Val Pro Met Ile Ala Trp Pro Leu Tyr Ala Glu
       370                 375                 380
   Gln Lys Met Asn Ala Ile Thr Leu Thr Asp Asp Leu Lys Val Ala Leu
   385                 390                 395                 400
   Arg Pro Lys Val Asn Glu Asn Gly Leu Ile Asp Arg Asn Glu Ile Ala
                   405                 410                 415
   Arg Ile Val Lys Gly Leu Met Glu Gly Glu Glu Gly Lys Gly Val Arg
               420                 425                 430
   Ser Arg Met Lys Asp Leu Lys Asp Ala Ser Ala Lys Val Leu Ser His
           435                 440                 445
   Asp Gly Ser Ser Thr Lys Ala Leu Ser Thr Val Ala Gln Lys Trp Lys
       450                 455                 460
   Ala His Lys Asn Tyr
   465

## Description

The present invention discloses a glycosyltransferase for producing an aroma glycoside, a nucleic acid molecule encoding the glycosyltransferase, a vector comprising a DNA sequence encoding the glycosyltransferase, a host cell containing or transfected with the nucleic acid molecule or the vector, a transgenic plant comprising the nucleic acid molecule or the vector, and a method of forming at least one aroma glycoside, wherein an aglycone is covalently linked to a sugar donor comprising contacting the aglycone with the sugar donor and the glycosyltransferase.

Described is the full application of biotechnologically produced inactive aroma- and fragrance/scent precursors in new concentration ranges beyond those of natural aroma glycoside sources, which can be activated by external triggers like enzymes, temperature and pH, and then release the aroma and the fragrance. In addition, described is the use of biotechnologically produced inactive fragrance precursors in every-day commodities which can be activated by external triggers like enzymes, temperature and pH. Products which - in addition to the inactive fragrance precursor - already include the necessary enzyme in e.g. a dry state for activation only need wetting/moistening by water to release the desired fragrance.

Flavorings and fragrances are used in the field of foods, hygiene and body care / personal hygiene in manifold ways. The flavorings and fragrances applied are often coming from natural sources, particularly plants. Many of these flavorings and fragrances are nowadays also produced by chemical synthesis or naturally (according to flavorings regulation (EG). No. 1334/2008 Art. 3(2)(c)) via biotechnology and are already accepted on the market in this form.

The quality of a flavoring or a fragrance is determined by its perceived taste and/or odor. This depends, among others, on the concentration of the added/inserted flavoring or fragrance. The taste or odor should be sensed as pleasant by the consumer and have a taste or smell in line with its application. Generally, however, only a limited number of flavorings and fragrances is used.

This is evident, for example, by recurring ingredients in deodorants, like geraniol (smell of roses), which is included in about 48% of such products. Variants of the products often are simply designed using finely tuned, special mixtures of well-known components.

In order to maintain fragrances for the consumer over a prolonged period of time, e.g. an 8 hours working day, generally high concentrations are applied, what leads to - apart of the odor aspect for the consumer, i.e. strong smell at the beginning, too weak smell at the end - higher costs for the producer due to a quantitatively higher usage of the fragrance.

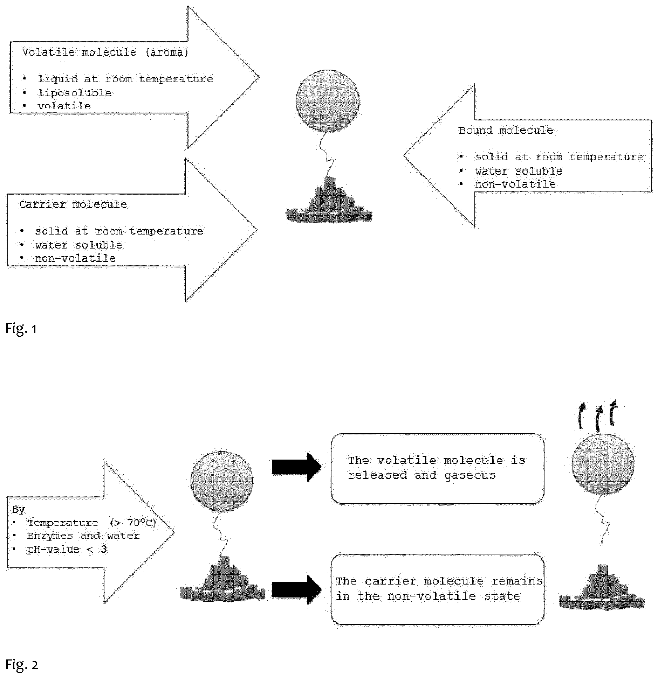

A surprisingly simple, natural solution for releasing bound flavorings is found in fruits and other organs of several plants. Many secondary metabolites of plant origin, i.e. substances which are only present in specific plant groups, are stored in the plant in the form of glycosides, i.e. bound to a sugar (Fig. 1). In this way the substances are stabilized and commonly biologically inactive, are not volatile and water soluble. Flavorings and fragrances bound to a sugar are not any more active as flavorings or fragrances in this form - as glycosides.

The release of such bound aromas takes place in nature only by dissolving cellular structures by released enzymes, e.g. glycosidases from the vacuole, or through microbial enzymes which release the actual active agent from its carrier molecule glucose (Fig. 2). Apart from the enzymatic cleavage the release of the aroma agent can also take place by heating or changing the pH value.

Basic examples are the aroma glycosides of grape vines. The terpenes released from glycosides are natural attractants promoting the consumption of the fruit and therefore the spreading of the seeds. They are also important components of the wine aroma after their release by fermentation.

A few hundred aroma glycosides are present in plants. They are produced in the cell by so-called glycosyltransferases which transfer to the respective aroma molecule a sugar residue, often glucose, i.e. glycosylate it. This stabilizes the compound in water-soluble and non-volatile form.

The glycosyltransferases often convert various flavorings and fragrances, but often prefer one substance class. The multitude of flavorings and fragrances occurring is accompanied by a likewise multitude of glycosyltransferases whose genes can be isolated from exactly these plants in which such a glycoside of interest is contained. These genes can be introduced into GRAS-organisms (generally recognized as safe / genetically regarded as safe) like yeast or distinct *E. coli* strains and thus enable these to glycosylate almost arbitrary substances.

The specific glycosyltransferase genes have to be isolated and cloned for a specific biotechnological application. But it is usually quite difficult to identify exactly the glycosyltransferase of interest in a specific plant with an interesting glycoside as the desired glycosyltransferase is only one of hundreds present in every plant.

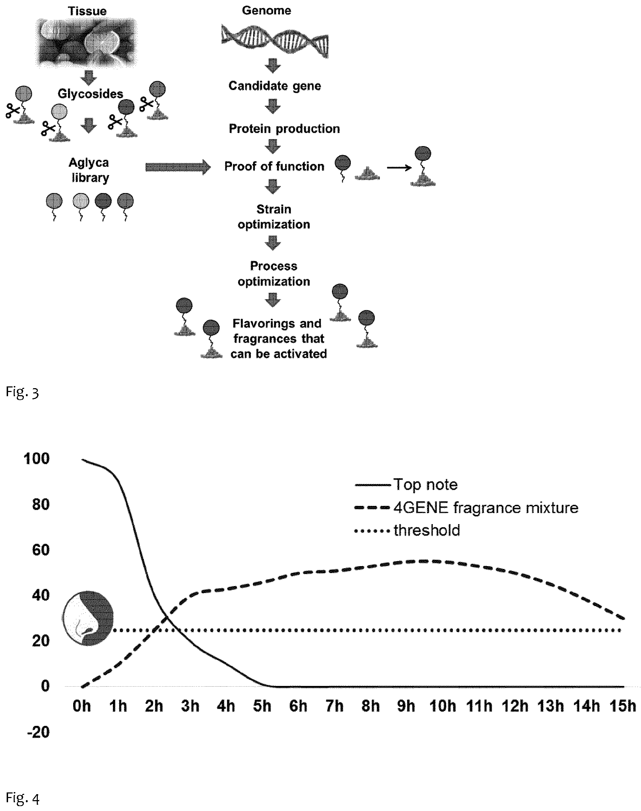

In EP -A-2960339 published on 30.12.2015, this problem is solved by developing a test procedure that enables a fast identification of a desired glycosyltransferase gene from a plant with a glycoside relevant for aromas (Fig. 3). A so-called "aglycone-library" of a plant having an aroma/flavoring and/or fragrance glycoside of interest for aroma houses is created thereby by first isolating all glycosides thereof as an overall mixture and then cleaving their sugar residues. Candidate genes for respective glycosyltransferases, e.g. from genome data or sequencing, are used to produce the respective enzyme in bacteria or yeast and thereby treat the whole library and test it. Only the natural target molecule of this gene / enzyme, e.g. the flavoring of fragrance, is glycosylated again und can thereby be easily isolated and identified from the bulk of remaining aglycons. In this way the gene / enzyme is identified and can be transferred to production strains and thereby used of production of aromas that can be activated (see, for example, the sequences of EP A-2960339 and example 8, biotechnological production of furanon-glycosides using glycosyltransferases).

Up to now aroma / flavoring and fragrance glycosides can only be produced by reverse enzymatic hydrolysis with glycosidases. This biocatalysis, which mostly produces α-glucoside, is inefficient, however, and the products are therefore expensive. A chemical synthesis would also be possible.

This is, however, very complex - comprising protective group chemistry, anhydrous conditions, heavy metal catalysts und double cleaning - and thus also expensive. Both these production methods have until now not lead to the commercial availability of higher amounts (kg-quantity) of aroma glycosides / flavoring and fragrance glycosides.

Several methods exist to release the aroma, respectively the odor, from chemically inactived, bound or encapsulated flavorings and fragrances just by a mechanism, see Table 1, which also shows already a comparison of these methods compared to the present method. In general the purpose is either to modify volatile flavorings and fragrances in such a way that their physical-chemical properties are changed, to bind them to various matrices or to encapsulate them, and they thus not evaporate anymore. Afterwards it has to be possible or release them by simple means. Alcohols (e.g. citronellol), aldehydes and ketones (e.g. vanillin), esters and lactones (e.g. cumarin) can be used for this purpose. Methods for the release then are temperature, oxidation, light, enzymes, microorganism and hydrolases as well as changes of the pH-value (Herrmann, 2007, Angew. Chem. Int. Ed. Engl. 46, 5836-5863).

**Table 1: Strength of the technologies for important characteristics**

| | Products according to the invention | Other chemical precursor systems | Encapsulation | Binding to carriers |
|---|---|---|---|---|
| Chemical release | +++ | ++ | ++ | |
| Biological release | +++ | | | |
| Physical release | +++ | ++ | ++ | ++ |
| Good solubility in water | +++ | | | + |
| Storage possible over long time | +++ | ++ | | |
| Without impurities in foods | +++ | ++ | | |
| Additional functions | +++ | | | |

| | | | | |
|---|---|---|---|---|
| +++ = very good, ++ = good, + = satisfactory | | | | |

A known example is the addition of digeranyl succinate or hexarose (geranyl palmitate) to softeners so that geraniol and nerol (rose fragrance) are released by the lipase - a hydrolase - present in the detergent for solving fats.

Volatile compounds are also encapsulated into wrappings of different materials and different dimensions ("micro-, macro- and nanoencapsulation"), are thus not volatile anymore and can be released by different methods: solvation in water, temperature, mechanical forces, enzymes, chemical reactions and pH change. Here the methods of release are not related to the flavoring or fragrance, though, but to the capsule material. Generally the applicability of these methods is limited to products with high prices as the encapsulation, respectively binding to carriers, is complex and thus expensive. Furthermore, the flavorings and fragrances can diffuse through the capsule material at longer storage times and thus be lost. This technique is less suitable for additives to foods, as the capsule material would reach the food as well (Feng et al., 2009, Recent Pat Food Nutr. Agric. 1, 193-202).

In addition, methods are known in which flavorings and fragrances are bound to carrier materials, e.g. cyclodextrins, starch, and/or proteins. Here, methods like extrusion, spray-drying, agglomeration, e.g. wet agglomeration in mixers or fluidized bed agglomeration, or in recent days especially fluidized bed - spray granulation, are applied. An important feature herein is that the flavorings and fragrances are subject to a depot effect, i.e. they can be defined, but not be controlled during the further process of release as the desorption and diffusion rate is fixed. Like in encapsulation also here an additional material is needed as carrier, so that also here contaminants/impurities are needed (Feng et al., 2009, Recent Pat Food Nutr. Agric. 1, 193-202). WO 2006/087370 A1 discloses the use of aroma glycosides as flavor or fragrance ingredients in food, feed, cleaning liquid or a washing powder products.

In Koeduka et al., "Enhancement of production of eugenol and its glycosidases in transgenic aspen plants via genetic engineering", Biochem Biophys Res Comm, 436, 1, pp. 73-78, 2013, a study attempted to modify the production of eugenol and its glycosides by introducing petunia coniferyl alcohol acetyltransferase (PhCFAT) and eugenol synthase (PhEGS) into hybrid aspen is disclosed.

Further, Gosch C. et al, "Cloning and heterologous expression of glycosyltransferases from Malus x domestica and Pyrus communis, which convert phloretin to phloretin 2-O-glucoside (phloridzin)", Plant Sci, 178, 3, pp.299-306, 2010 describes cloning and characterization of five novel glycosyltransferases with low amino acid sequence identities (34.1-46.3%) from apple (clones UGT71A15 and UGT71K1) and pear (clones UGT71A16, UGT71K2 and UGT88F2), which are able to glycosylate phloretin to phloridzin in vitro.

Y.M. Tikunov et al., "Non-smoky glycosyltransferase 1 prevents the release of smoky aroma from tomato fruit", Plant Cell, 25, 8, pp. 3067-3078, 2013 refers to the use of reverse genetics approaches to show that the NSGTi-mediated glycosylation is the molecular mechanism underlying the major quantitative trait locus for smoky aroma.

T. Louveau et al.; "Predicting the substrate specificity of a glycosyltransferase implicated in the production of phenolic volatiles in tomato fruit", FEBS J, 278, 2, pp. 390-400, 2011 discloses the use of a homology-based screen to identify the gene SIUGT5, which shows specificity towards a range of substrates.

Furthermore, a predicted sequence can be found in "PREDICTED: anthocyanidin 3-O-glucosyltransferase 2-like [Fragaria vesca subsp. vesca], database accession no. XP_004294260.1, and a further sequence can be found under database accession no. D7UCM3.

Yar-Khing Yauk et al., "Manipulation of flavor and aroma compound sequestration and release using a glycosyltransferase with specificity for terpene alcohols", Plant J, 80, 2, pp. 317-330, 2014 refer to a screen of UGTs expressed in kiwi-fruit for identifying the gene AdGT4.

### Summary of the invention

The invention is defined by the appended claims. This application shows the feasibility of the use of flavoring and fragrance precursors in bigger measures. The provision of a broad variety of such substances is not known until now, and this is enabled with the new glycosyltransferase method. The present inventors found that the glycosyltransferase of the present invention enables for the first time the production of aroma glycosides in amounts that are feasible for biotechnological production thereof, thus enabling the use of aroma glycosides as additives in products or compositions in amounts that could not be achieved until now. Although the present glycosyltransferase only differs from natural glycosyltransferases in its coding nucleic acid sequence in a few nucleic acids, making up a change in only a few amino acids, it achieves a remarkably improved production of aroma glycosides and, apart from that, also is able to form aroma glycosides that could not be produced until now. Described is a product or composition comprising at least one aroma glycoside comprising an aglycone component and at least one sugar component, wherein the concentration of the at least one aroma glycoside is higher than its concentration in natural products or compositions.

Part of the invention is a glycosyltransferase for producing an aroma glycoside coded by a nucleic acid sequence that
a) comprises the sequence of SEQ-ID2.

Described is (A) a glycosyltransferase for producing an aroma glycoside coded by a nucleic acid sequence that
a) comprises the sequence of SEQ-ID1 or SEQ-ID3; or
b) comprises a sequence that is at least 90%, preferably at least 95%, more preferably at least 98%, identical to SEQ-ID1, SEQ-ID2, or SEQ-ID3; or
c) comprises a part of the sequence of SEQ-ID1, SEQ-ID2, or SEQ-ID₃, wherein said part of the sequence of SEQ-ID1, SEQ-ID2, or SEQ-ID₃ codes at least 50, preferably at least 80, more preferably at least 100, more preferably at least 200, amino acids in length; or
d) comprises a sequence that is at least 90%, preferably at least 95%, more preferably at least 98%, identical to a part of the sequence of SEQ-ID1, SEQ-ID2, or SEQ-ID₃, wherein said part of the sequence of SEQ-ID1, SEQ-ID2, or SEQ-ID₃ codes at least 50, preferably at least 80, more preferably at least 100, more preferably at least 200, amino acids in length.

A further aspect of the invention relates to a nucleic acid molecule encoding a glycosyltransferase according to the invention, wherein said nucleic acid molecule is a DNA molecule, particularly a cDNA molecule. Described is also (B) a nucleic acid molecule encoding glycosyltransferase as given in the above (A), wherein said nucleic acid molecule is a DNA molecule, particularly cDNA molecule.

According to a further aspect of the invention, a vector comprising a DNA sequence encoding a glycosyltransferase according to the invention is disclosed. Described is (C) a vector comprising a DNA sequence encoding a glycosyltransferase according to the above (A).

In addition, a host cell containing or transfected with the nucleic acid molecule according to the invention or the vector according to the invention is disclosed as a further aspect of the present invention. Described is (D) a host cell containing or transfected with the nucleic acid molecule according to the above (B) or the vector according to the above (C).

A further aspect of the present invention relates to a transgenic plant comprising the nucleic acid molecule of the invention or a vector of the invention. Described is (E) a transgenic plant comprising the nucleic acid molecule according to the above (B) or the vector according to the above (C).

A further aspect of the present invention is a method of forming at least one aroma glycoside, wherein an aglycone is covalently linked to a sugar donor comprising contacting the aglycone with the sugar donor and the glycosyltransferase of the invention under conditions appropriate for the transfer of the sugar group of said sugar donor. Described is a method of forming at least one aroma glycoside, wherein an aglycone is covalently linked to a sugar donor comprising contacting the aglycone with the sugar donor and the glycosyltransferase according to the above (A) under conditions appropriate for the transfer of the sugar group of said sugar donor. Described is a method of producing at least one aroma glycoside, said method comprising the steps of:
- culturing or growing the host cell of the invention or the transgenic plant of the invention or the host cell according to the above (D) or the transgenic plant according to the above (E) ; and
- collecting from said host cell or transgenic plant said aroma glycoside.

Also described is a product produced by the inventive methods.

Also the use of an aroma glycoside in a concentration higher than its concentration in natural products or compositions is described.

Further embodiments are disclosed in the dependent claims and can be taken from the following description and examples.

The invention will be further described in detail in the following with reference to the figures and particular embodiments thereof, without being limited thereto.

In the figures the following is shown:
Fig. 1 shows an abstract schematic of the composition of the glycosides used in the present invention.
Fig. 2 depicts schematically the release of the flavoring or fragrance from the aroma glycosides.
Fig. 3 shows the use of an aglyca library to produce aroma glycosides
Fig. 4 discloses the data of a fragrance precursor mixture ("4GENE Duftstoffgemisch", 4GENE fragrance mixture) in body deodorant used in example 1.
Fig. 5 shows pictures A 1: Photos of the breads from baking test 1 in Example 5 after the baking process: 1.1 blind (negative) sample; 1.2 a-c: sample blanks; 1.3 control; 1.4 recovery
Fig. 6 shows pictures A 2: Photos of the breads from baking test 2 in Example 5 after the baking process: 2.1 blind (negative) sample; 2.2 a-c: sample blanks; 2.3 recovery
Fig. 7 shows pictures A3: Photos of the breads from baking test 3 in Example 5 after the baking process: 3.1 blind (negative) sample; 3.2 a-c: sample blanks; 3.3 recovery
In Fig. 8 picture A4 is depicted, showing a 50 g-blank bread for sensory testing in Example 5
Fig. 9 shows Picture A5 of the Triangle test in Example 5 - sensory vessels with bread samples (pictures processed)
In Fig. 10 artificial aromatic flowers that can be activated of Example 11 are shown.
In Fig. 11 the determination of the enzyme activity of FaGTs1-5 and FaGTs1-13 in Example 11 is shown.

### Detailed description of the present invention

All ranges disclosed herein are to be considered to be supplemented by the term "about", unless clearly defined to the contrary or otherwise clear from the context.

Described is a product or composition comprising at least one aroma glycoside comprising an aglycone component and at least one sugar component, wherein the concentration of the at least one aroma glycoside is higher than its concentration in natural products or compositions.

A "glycoside", as used herein, is a molecule in which a sugar (the "glycone" part or "glycone component" of the glycoside) is bonded to a non-sugar (the "aglycone" part or "aglycone component") via a glycosidic bond. Accordingly, a glycoside may consist of a sugar as glycone component linked through its anomeric carbon atom to e.g. the hydroxy group of an alcohol (chemical structure R-OH) as aglycone component. For example, in the glycoside linaloyl β-D-glucoside, the glycone component glucose is linked to the aglycone component linalool.

A glycoside can be produced by carrying out a reaction in which an aglycone component is mixed under appropriate conditions with a sugar donor in the presence of a glycosyltransferase as enzymatic catalyzer.

Alternatively, a glycoside can be produced by culturing or growing a host cell or transgenic plant expressing a glycosyltransferase according to the invention when adding the respective aglycone and sugar. During culture/growth, such a host cell or transgenic plant will generate glycosides.

The glycoside(s) generated in such a host cell or transgenic plant can subsequently be collected from said host cell or transgenic plant by standard methods of extraction and/or chromatography (such as solvent extraction, solid phase extraction and reversed phase chromatography).

The aroma - and thus the aglycone - are not particularly restricted and can be any that is available, either from natural sources or produced synthetically. According to certain aspects of the description, the aroma glycoside is produced from a natural source, e.g. in a plant or in microorganisms. The aroma glycoside can then be introduced into the product or composition, thus producing a man-made product or composition, which is not particularly restricted. Several possible products and compositions will be described in the following, including, but not limited to, cosmetic products, e.g. deodorants, shower gels, body lotions, skin creams, shampoos, lipsticks, skin whitenings, and tooth pastes; hygienic products, e.g. diapers, sanitary pads, shoe soles, litter bins, cat toilets, and pet beds; food products, e.g.. bakery products, frozen foods, tea, coffee, beverages, chewing gum, sweets, pills against bad breath, deodorant candies, flavor enhancers, blocking/inhibitors of negative flavors, blocking bitter tastes / bitter taste inhibitors, and taste modulators; pharmaceutical products; fragrances; clothing and textiles and products related thereto, e.g. fabric softeners, and impregnations; household products, e.g. scented candles and cards, cigarettes, air fresheners, artificial flowers and plants, and adhesive strips; products in the construction sector, e.g. wood preservatives, wallpapers, mold-fightings /mold control, and odor colors; insect repellents, e.g. skin creams (topical), candies (orally), and candles; and products for electronic devices, e.g. odor TVs / 4D TVs, and smartphone gadgets. Also concentrates of the aroma glycosides can be produced that can be then used for producing any of the products or compositions.

A variety of the aroma glycosides, being aroma and odor precursors, that can be activated and produced biotechnologically by the glycosyltransferase according to the invention or the one described in the above (A) are described below. A basis thereof are aromas like flavorings and fragrances of natural origin that can then be glycosylated, with a small selection of the most important flavorings and fragrances presented in table 2, although not limited thereto; generally, all alcohols of the list of the European union can also be envisioned ("COMMISSION IMPLEMENTING REGULATION (EU) No 872/2012 of 1 October 2012 adopting the list of flavoring substances provided for by Regulation (EC) No 2232/96 of the European Parliament and of the Council, introducing it in Annex I to Regulation (EC) No 1334/2008 of the European Parliament and of the Council and repealing Commission Regulation (EC) No 1565/2000 and Commission Decision 1999/217/EC") - source: http://eur-lex.europa.eu/legal-content/EN/TXT/?uri=CELEX:32012R0872; further, also glycosylated terpenes as disclosed in http://www.ebi.ac.uk/chebi/searchld.do?chebild=61777 can be produced.

Also derivatives, isomers of the respective substances are included in the list of possibilities.

The aroma can be at least one selected from the list of compounds consisting of eugenol, citronellol, thymol, carvacrol, furaneol, 1-hexanol, homofuraneol, norfuraneol, menthol, raspberry ketone, maltol, ethylmaltol, 2-furfurylthiol, 2-methyl-3-furanthiol, 3-mercapto-2-pentanon, 1-octen-3-01, sotolon, 4-mercapto-4-methylpentan-2-on, (3-methylthio)-1-propanol, and phenylethanol, e.g. from thymol, carvacrol, furaneol, homofuraneol, norfuraneol, raspberry ketone, maltol, ethylmaltol, 2-furfurylthiol, 2-methyl-3-furanthiol, 3-mercapto-2-pentanon, sotolon, 4-mercapto-4-methylpentan-2-on, and (3-methylthio)-1-propanol, e.g. from carvacrol, norfuraneol, raspberry ketone, 2-furfurylthiol, 2-methyl-3-furanthiol, 3-mercapto-2-pentanon, sotolon, 4-mercapto-4-methylpentan-2-on, and (3-methylthio)-1-propanol. Described are glucosides thereof.

The aroma glycoside can comprise an aglycone precursor selected from the group consisting of odorous substances, flavoring agents, essences, perfume oils, perfumes, aromatic substance compositions, and fragrance mixtures.

**Table 2: Selection of flavoring and fragrance precursors that can be produced; the following flavorings and fragrances can be glycosylated, e.g. glucosilated, and thus be converted to precursors**

| Flavoring or fragrance | Preferred for low odor threshold | Flavoring or fragrance | Preferred for low odor threshold |
|---|---|---|---|
| geraniol | x | heptyl alcohol | x |
| nerol | x | linalool | x |
| citronellol | x | menthenethiol | x |
| furaneol | x | octalactone | x |
| thymol | x | octenol | x |
| carvacrol | | propanethiol | x |
| eugenol | x | raspberry ketone ("H imbeerketon") | x |
| phenyl ethanol | | sotolon, caramel furanone | x |
| vanillin | | terpineol | x |
| menthol | | 10-undecenol | x |
| hexenol | x | hydroxycitronellol | |
| anisole | x | dihydromyrcenol | |
| butanethiol | x | borneol | |
| decalactone | x | hesperetin | |
| ethylguaiacol | x | raspberry ketone | |
| farnesol | x | homofuraneol | |
| furfuryl alcohol | x | norfuraneol | |
| furfuryl mercaptan | x | maltol | |
| guaiacol | x | ethyl maltol | |

| | | | |
|---|---|---|---|
| In Table 2, "x" means that the compound is preferred. | | | |

Also the sugar component is not particularly limited. Different sugars can be used for producing the precursors, as disclosed in e.g. table 3, although not being limited thereto.

Table 3: Selection of sugars: instead of using glucose (glucosides) also other sugars can be used, the resulting precursors will then generally be called glycosides or be named according to the respective sugar; list of sugars (selection):
Glucose,
Arabinose,
Xylose,
Rhamnose,
Apiose,
Galactose

The term that the concentration of the at least one aroma glycoside is higher than its concentration in natural products or compositions means that the concentration of the aroma glycoside in the product or composition is higher than a concentration in which it can be observed in a natural product, particularly any natural product.

The concentrations can thereby vary according to the aglyca and sugar component. For example, furaneol can be contained in raspberries in concentrations of about 0.8 mg/kg fruit naturally.

The herein described products and compositions can comprise glycosides of natural aroma agents.

The concentration of the at least one aroma glycoside in the product or composition can be 1-10000 ppm, preferably 2 - 10000 ppm, further preferably 10 - 1000 ppm, more preferably 20 - 200 ppm, even further preferably 50 - 150 ppm, particularly preferably about 100 ppm. If more than one aroma glycoside is contained in the product or composition, more than one, e.g. each, can have a concentration in the respective ranges, although it is sufficient if at least one aroma glycoside has a concentration in the above ranges.

The aroma glycoside can be produced by a biotechnological process. Genes of at least one glycosyltransferase can be isolated from at least one plant, particularly from a plant in which a glycoside of interest is contained, and then introduced into an organism, particularly GRAS-organisms (generally recognized as safe / genetically regarded as safe), e.g. microorganisms like yeast or distinct *E. coli* strains, and one or more aroma glycoside can be produced using these organisms by known procedures, e.g. culturing in presence of educts for producing the aroma glycoside.

The product or composition can further comprise at least one inactive hydrolase, e.g. a glycosidase, e.g. a β-glucosidase. This hydrolase can then be activated in the product or composition, e.g. by water when the hydrolase is present in a dry state, so that the aroma is released.

The product or composition can further also comprise at least one inactive hydrolase, e.g. a glycosidase, e.g. a β-glucosidase, and at least one further inactive enzyme, e.g.when the aglycone is an alcohol and/or a thiol, e.g. an oxidase when the aglycone is an alcohol.

The further inactive enzyme can be e.g. also a dry enzyme that is activated upon contact with water, thus enabling a delayed functionality of the enzyme. Not only the additional enzyme can be inactive, but also the at least one hydrolase, e.g. a glycosidase, e.g. a β-glucosidase, thus enabling a prolonged and/or delayed release of the aroma upon cleavage of the aroma glycoside by the hydrolase, e.g. glycosidase. Particularly useful are β-glucosidases as the glycosyltransferases as they can particularly catalyze the formation of β-glucosides that can be cleaved by the human microbiome. α-glucosides produced biotechnologically up to now, which further carry several sugar units, can only be released intricately using sequential reactions and with special glucosidases.

The presence of a further enzyme can enable the formation of a different aroma from the aroma first released, e.g. the formation of an aldehyde from an alcohol, wherein both compounds function as aromas, e.g. flavors and/or fragrances, leading to the possibility of producing mixtures of aromas that can be controlled by the release and reaction of the first aroma. Described is also the use of thiols, which can be synthesized using the glycosyltransferases, as they can be perceived in far lower amounts, and also can be oxidized to achieve new aromas. Although oxidases are exemplified herein as further enzymes, other enzymes can be present. In the case of alcohol aglycones an oxidase is preferred as further enzyme, though.

The product or composition can also contain more than one aroma glycoside in combination/mixtures, wherein the aroma glycosides can release the aroma either by the same or different procedures after activation. It is thus possible that different aromas are released at the same time, leading to a mixture of aromas with a particular note, or that different aromas are released at different times, leading to arrangements of aromas, e.g. fragrances that change over the course of time. It is also not excluded that the same aroma is coupled to different sugars which are activated by different procedures, so that the aroma can be released from different sources over an even further prolonged period of time.

The product or composition can further comprise at least one selected from the group consisting of odorous substances, flavoring agents, essences, perfume oils, perfumes, aromatic substance compositions, and fragrance mixtures, etc. This way it is possible that the product or composition first exerts the fragrance or flavor of the odorous substances, flavoring agents, essences, perfume oils, perfumes, aromatic substance compositions, and/or fragrance mixtures, etc., whereas the same fragrance or flavor or a different one can further be maintained upon release of the aroma from the aroma glycosides, thus enabling the perception of the aroma from the beginning, even without cleavage of the aroma glycoside, and for a long time upon cleavage of the aroma glycoside. Also mixes of different aromas, one coming from the aroma glycoside, can be achieved.

It is not excluded that the aroma glycoside can have a further function in the product or composition, replacing other additives. One example is the usual addition of digeranly succinate or hexarose (geranyl palmitate) to softeners so that geraniol and nerol (rose fragrance) are released by the lipase - a hydrolase - present in the detergent for solving fats. Here, also the aroma glycosides, respectively flavoring and fragrance glycosides can be used in place of digeranly succinate or hexarose, which also are activated by further hydrolases (cellulases) present in detergents. Due to their bipolar structure (sugar and non-sugar component) these additionally have the advantage that they can be used as surfactant in their inactive form. Other surfactants thus would be required in decreased amounts, leading to savings for the detergent producers.

According to a first aspect, the present invention relates to a glycosyltransferase for producing an aroma glycoside coded by a nucleic acid sequence that
a) comprises the sequence of SEQ-ID2.
   Described is a glycosyltransferase for producing an aroma glycoside coded by a nucleic acid sequence that comprises the sequence of SEQ-ID1 or SEQ-ID3; or
b) comprises a sequence that is at least 90%, preferably at least 95%, more preferably at least 98%, further preferably at least 99%, identical to SEQ-ID1, SEQ-ID2, or SEQ-ID3; or
c) comprises a part of the sequence of SEQ-ID1, SEQ-ID2, or SEQ-ID₃, wherein said part of the sequence of SEQ-ID1, SEQ-ID2, or SEQ-ID3 codes at least 50, preferably at least 80, more preferably at least 100, more preferably at least 200, amino acids in length; or
d) comprises a sequence that is at least 90%, preferably at least 95%, more preferably at least 98%, further preferably at least 99%, identical to a part of the sequence of SEQ-ID1, SEQ-ID2, or

SEQ-ID3, wherein said part of the sequence of SEQ-ID1, SEQ-ID2, or SEQ-ID3 codes at least 50, preferably at least 80, more preferably at least 100, more preferably at least 200, amino acids in length.

Outside the scope of the invention, the glycosyltransferase for producing an aroma glycoside as used in a product or composition above coded by a nucleic acid sequence can comprise an amino acid sequence resulting from SEQ-ID1, or SEQ-ID₃, or a part of SEQ ID NO:1, SEQ ID NO:2 or SEQ ID NO:3, wherein the part constitutes at least 50, preferably at least 80 or most preferably at least 200 amino acids in lengths; or comprises an amino acid sequence which is at least 90%, preferably at least 95% or most preferably at least 98% identical to the amino acid sequence resulting from SEQ-ID1, SEQ-ID2, or SEQ-ID₃, or a part thereof, wherein the part constitutes at least 50, preferably at least 80 or most preferably at least 200 amino acids in lengths.

The glycosyltransferase according to the invention for producing an aroma glycoside coded by a nucleic acid sequence that comprises the sequence of SEQ-ID2 corresponds to a glycosyltransferase for producing an aroma glycoside having an amino acid sequence that comprises the sequence of SEQ-ID5, which is thus disclosed as well.

Described is a glycosyltransferase for producing an aroma glycoside as used in a product or composition above having an amino acid sequence that
a) comprises the sequence of SEQ-ID4, or SEQ-ID6; or
b) comprises a sequence that is at least 90%, preferably at least 95%, more preferably at least 98%, further preferably at least 99%, identical to SEQ-ID4, SEQ-ID5, or SEQ-ID6; or
c) comprises a part of the sequence of SEQ-ID4, SEQ-ID5, or SEQ-ID6, wherein said part of the sequence of SEQ-ID4, SEQ-ID5, or SEQ-ID6 is at least 50, preferably at least 80, more preferably at least 100, more preferably at least 200, amino acids in length; or
d) comprises a sequence that is at least 90%, preferably at least 95%, more preferably at least 98%, further preferably at least 99%, identical to a part of the sequence of SEQ-ID4, SEQ-ID5, or

SEQ-ID6, wherein said part of the sequence of SEQ-ID4, SEQ-ID5, or SEQ-ID6 is at least 50, preferably at least 80, more preferably at least 100, more preferably at least 200, amino acids in length.

Herein, the amino acid sequences SEQ-ID4, SEQ-ID5, or SEQ-ID6 are coded by the nucleic acid sequences SEQ-ID1, SEQ-ID2, or SEQ-ID₃, respectively.

The glycosyltransferase can be a small molecule glycosyltransferase. A small molecule glycosyltransferase is a glycosyltransferase that catalyzes the transfer of a monosaccharide moiety from a sugar donor to a small molecule as acceptor molecule. A small molecule is a molecule that has a molecular weight below 1 500 Dalton, preferably below 1 000 Dalton.

According to certain embodiments, the present invention refers to a glycosyltransferase coded by a nucleic acid sequence having the sequence of SEQ-ID2 or has the amino acid sequence of SEQ-ID5. Described is a glycosyltransferase coded by a nucleic acid sequence having the sequence of SEQ-ID1 or SEQ-ID₃ or has the amino acid sequence of SEQ-ID4 or SEQ-ID6. This is meant to designate that the nucleic acid sequence, respectively amino acid sequence, of said glycosyltransferase consists of said certain nucleic acid sequence, , respectively amino acid sequence.

Glycosyltransferases having a nucleic acid sequence comprising SEQ-ID1 (i.e. the sequence of FaGTs1-5) or SEQ-ID2 (i.e. the sequence of FaGTs1-13) or SEQ-ID₃ (i.e. the sequence of VvdGT13) or a related nucleic acid sequence can be obtained by standard methods of recombinant DNA technology.

According to certain embodiments, the glycosyltransferase is capable of catalyzing formation of a glycoside in which a sugar is linked to an aglycone through a β-D-glycosyl linkage.

According to a further aspect the present invention relates to a nucleic acid molecule encoding a glycosyltransferase of the invention, wherein said nucleic acid molecule is a DNA molecule, particularly a cDNA molecule. The present invention also refers to a cRNA molecule based on the DNA molecule of the invention. Described are a nucleic acid molecule encoding a glycosyltransferase described above, wherein said nucleic acid molecule is a DNA molecule, particularly a cDNA molecule, and a to a cRNA molecule based on such DNA molecule.

According to another aspect, the present invention refers to a vector comprising a DNA sequence encoding a glycosyltransferase of the present invention. Described is a vector comprising a DNA sequence encoding a glycosyltransferase as described above. Construction of a vector may be performed using e.g. a restriction enzyme, ligase etc. according to a standard method known in the art.

A further aspect of the present invention relates to a host cell containing or transfected with the nucleic acid molecule or the vector according to the invention. Described is a host cell containing or transfected with the nucleic acid molecule or the vector as described above.

As host cell transfected with the nucleic acid molecule as described above, the cell of a prokaryotic or eukaryotic organism or the prokaryote or eukaryote itself may be used. Bacteria, for example, commonly used hosts such as bacteria belonging to genus Escherichia such as Escherichia coli can be used as the prokaryotic organism, although also other prokaryotes can be used. Alternatively, a cell of a lower eukaryotic organism such as eukaryotic microorganisms including, for example, yeast (e.g. Saccharomyces cerevisiae) or fungi like Aspergillus oryzae and Aspergillus niger can be used. Animal cells or plant cells also can be used as a host. Examples of animal cells that can be used include cell lines of mouse, hamster, monkey, human, etc., as well as insect cells such as silkworm cells and adult silkworm per se.

A host cell being transfected refers to a situation where foreign DNA is introduced into the cell. A transfected host cell may be stably transfected, i.e. foreign DNA is introduced and integrated into the genome of the transfected cell. Alternatively, a transfected host cell may be transiently transfected, wherein the foreign DNA fails to integrate into the genome of the transfected cell.

The transformation of a host with a nucleic acid molecule or a vector can be performed according to standard methods.

According to a further aspect, the present invention relates to a transgenic plant comprising a nucleic acid molecule or a vector according to the present invention. Described is a transgenic plant comprising a nucleic acid molecule or a vector as described above. The term "transgenic plant" refers thereby to a plant that has a heterologous gene, i.e. a gene not from its natural environment, integrated into its genome and that transmits said heterologous gene to its progeny. For example, a heterologous gene includes a gene from one species introduced into another species. According to certain embodiments, a heterologous gene also includes a gene native to an organism that has been altered in some way (e.g., mutated, added in multiple copies, or linked to non-native regulatory sequences).

Another aspect of the present invention is directed to a method of forming at least one aroma glycoside, wherein an aglycone is covalently linked to a sugar donor comprising contacting the aglycone with the sugar donor and a glycosyltransferase of the invention under conditions appropriate for the transfer of the sugar group of said sugar donor. Described is a method of forming at least one aroma glycoside, wherein an aglycone is covalently linked to a sugar donor comprising contacting the aglycone with the sugar donor and a glycosyltransferase as described above under conditions appropriate for the transfer of the sugar group of said sugar donor. These appropriate conditions depend, among others, on the aglycone, the sugar donor and the glycosyltransferase, particularly its enzymatic activity, and thus also e.g. the temperature and pH, and can be suitable set once the compounds are determined. Said method can be an *in vitro* method which, preferably, does not involve any steps carried out *in vivo.* Said method can be an *in vivo* method carried out in a host cell or transgenic plant as defined above. According to certain embodiments, only one aroma glycoside is formed in this method. According to certain embodiments, the method comprises the steps of culturing or growing a host cell of the invention or a transgenic plant of the invention; and collecting from said host cell or transgenic plant said aroma glycoside.

Described is a method of producing at least one aroma glycoside, said method comprising the steps of:
- culturing or growing a host cell or a transgenic plant of the invention or as described above; and
- collecting from said host cell or transgenic plant said aroma glycoside.

The culturing or growing depends on the host cell or transgenic plant and can be suitably set by the skilled person, e.g. regarding nutrient addition, culture medium, growth conditions, etc. The collecting also depends on the type of host cell or transgenic plant and can be carried out using usual means like extraction, which also can be suitable set. Preferably, the aglycone is present in said host cell or transgenic plant during said culturing or growing said host cell or transgenic plant. Further preferably, the sugar donor is present in the culture medium used for culturing said host cell or in the water used for watering that transgenic plant. In certain embodiments, said culturing or growing said host cell is carried out in a bioreactor.

It is not excluded that more than one aroma glycoside is produced in a host cell or transgenic plant, which can be suitably carried out by selecting the respective aglycones and sugars.

According to certain embodiments, only one aroma glycoside is produced in a host cell or transgenic plant

Described is a product produced by the methods. The product herein can comprise also mixtures of aroma glycosides. Furthermore, the product can contain the aroma glycoside in a concentration higher than its concentration in natural products or compositions, particularly higher than its concentration when produced in a host cell or transgenic plant of the same species and/or type which is not according to the invention.

Further described is the use of an aroma glycoside in a concentration higher than its concentration in natural products or compositions, preferably in an artificial product or composition from the group consisting of cosmetic products, e.g. deodorants, shower gels, body lotions, skin creams, shampoos, lipsticks, skin whitenings, and tooth pastes; hygienic products, e.g. diapers, sanitary pads, shoe soles, litter bins, cat toilets, and pet beds; food products, e.g.. bakery products, frozen foods, tea, coffee, beverages, chewing gum, sweets, pills against bad breath, deodorant candies, flavor enhancers, blocking/inhibitors of negative flavors, blocking bitter tastes / bitter taste inhibitors, and taste modulators; pharmaceutical products; fragrances; clothing and textiles and products related thereto, e.g. fabric softeners, and impregnations; household products, e.g. scented candles and cards, cigarettes, air fresheners, artificial flowers and plants, and adhesive strips; products in the construction sector, e.g. wood preservatives, wallpapers, mold-fightings /mold control, and odor colors; insect repellents, e.g. skin creams (topical), candies (orally), and candles; and products for electronic devices, e.g. odor TVs / 4D TVs, and smartphone gadgets. The aroma glycoside used can be the aroma glycoside present in a product or composition described above. The aroma glycosides used in the product or composition described above can be used.

As already noted above, the aroma precursors described, e.g. the flavoring and fragrance precursors that can be activated open up many applications with their special properties, a few of which can be seen in Table 4.

The identified enzymes themselves can be marketed as products by, for example, adding them together with the sugar substrates to semi-finished products in order to neutralize volatile, bad tasting or smelling flavors or odors by transferring them to non-volatile precursors using the enzymatic reaction.

**Table 4 Selection of applications of the flavoring and fragrance precursors that can be activated; Use in:**

| | |
|---|---|
| Construction sector | Wood preservatives, Wallpapers, Building materials (mould control), Odor colors |
| Household | Scented candles, Batteries, Cigarettes, Scented cards, 4D television, Scent dispensers, Candles, Flowers, Paper flowers with absorbing stem |
| Hygiene | Diapers, Urinary bags, Shoe soles, Trash can, Swimming and bathing pools, Cat's litter box, Animal beds, Dog hygiene, Pheromone pills, Tampons, Latex gloves, Anti-mosquito-candies, Anti-mosquito-skin cream, Anti-mosquito agent, Repellents, Detergents |
| Cosmetics | Toothpaste, Shower gel, Body lotion, Skin cream, Hair gels, Skin lotion, Lipstick, Finger nails, Skin whitening |
| Food | Chewing gumi, Tea, Pills against bad breath, Coffee, Spices, Frozen goods (pizza, etc.), Pastries, Deodorant candy, Drinks, Flavor enhancers, Blocking of negative flavors, Bitter flavor inhibition, Taste modulation, |
| Textiles | Clothing |
| Electronics | Electric appliances, e.g. 4D television; sensors |

Certain specific products and uses are described in the following:
(1) Biotechnologically produced aroma glycosides, wherein they are inactive precursors of flavorings or fragrances, are applied in new concentration ranges beyond those of natural aroma glycoside sources, and can be appropriately activated by:
   a) biological mechanisms, e.g. addition of enzymes, e.g. β-glucosidase,
   b) physical mechanisms, e.g. an increase in temperature, e.g. above 70°C,
   c) chemical mechanisms, e.g. a pH change, e.g. below a pH of 3,
   d) aroma glycosides, characterized in that they are effective as taste modulators in uncleaved form, e.g. (and not limiting) as bitter-masking or sweet-enhancing agents, or also as flavorings of the perception *umami* or *kokumi.*
(2) Aroma glycoside according to (1), wherein
   a) the released aglycones are understood as e.g. odorous substances/scents, aroma agents, flavorings, perfume oils, odors/smells/fragrances, odorous substance compositions, odor compositions and the like.
   b) the aglycones are for example geraniol, nerol, citronellol, thymol, carvacrol, eugenol, phenyl ethanol, vanillin, menthol, hexenol, anisole, butanethiol, decalactone, ethylguaiacol, farnesol, furfuryl alcohol, furfuryl mercaptan, guaiacol, heptyl alcohol, linalool, methenthiol, octalactone, propanethiol, raspberry ketone, terpineol, 10-undecenol, hydroxycitronellol, dihydromyrcenol, borneol or hesperetin, or norfuraneol; particularly preferred are according to (1) a), b), c), but also according to d): furaneol, homofuraneol, caramel furanone, furanthiol, maltol, ethyl maltol, octenol, sotolon.
   b) organic alcohols are glycosylated, e.g. from the list of aroma agents approved in Europe (union list).
   c) they are terpene glycosides, e.g. glycosylated terpenoids with one or more hydroxy functions (CHEBI:61777 list).
   d) the aglycones are glycosylated with a proportion, e.g. molar proportion, of one or a multitude thereof with glucose, arabinose, xylose, rhamnose, apiose or galactose,
   e) they have additional functions, e.g. antimicrobial, antifungal effect, flavor enhancement, sweetening agent, repellent, detergent.
(3) Aroma glycoside according to (1), wherein they are produced by a biotechnological process, for example via glycosyltransferases, e.g. with glucosyltransferases
   a) with the nucleotide sequence SEQ-ID1 and the amino acid sequence resulting thereof, or an amino acid sequence which is at least 90%, preferably at least 95% or most preferably at least 98% identical to the amino acid sequence resulting from SEQ-ID1, or a part thereof, wherein the part constitutes at least 50, preferably at least 80 or most preferably at least 200 amino acids in lengths,
   b) with an amino acid sequence which is at least 90%, preferably at least 95% or most preferably at least 98% identical to the amino acid sequence resulting from SEQ-ID2, or a part thereof, wherein the part constitutes at least 50, preferably at least 80 or most preferably at least 200 amino acids in lengths,
   c) with the nucleotide sequence SEQ-ID₃ and the amino acid sequence resulting thereof, or an amino acid sequence which is at least 90%, preferably at least 95% or most preferably at least 98% identical to the amino acid sequence resulting from SEQ-ID3, or a part thereof, wherein the part constitutes at least 50, preferably at least 80 or most preferably at least 200 amino acids in lengths,
   d) which are for example a terpene glycosyltransferase, preferably a monoterpene glycosyltransferase,
   e) which for example bind an aglycone containing a hydroxy group with a sugar via a β-D-glykosyl-bond to a glycoside,
   f) which use for example UDP sugars as sugar source, e.g. UDP-glucose, UDP-arabinose, UDP-xylose, UDP-rhamnose, UDP-apiose, UDP-galactose,
   g) which for example glycosylate the aglycones according to (2).
(4) A vector with the DNA-sequence from (3), which codes a glycosyltransferase.
(5) A host cell containing or transfected with the nucleic acid molecule according to (3) or containing the vector according (4).
(6) The host cell according to (5), which produces a glycosyltransferase according to (3).
(7) A transgenic plant comprising a nucleic acid molecule according to (3) 6 or a vector according to (4).
(8) The transgenic plant according to (7), which produces a glycosyltransferase according to (3).
(9) Use of a glycosyltransferase according to (3) or a vector according to (4) or a host according to (5) - (6) or a transgenic plant according to (7) - (8) for producing aroma glycosides according to (1) - (2).
(10) Aroma glycose according to (1), wherein they are used for
   a) hygienic applications and products, e.g. body deodorant, diapers, urinary bags, shoe soles, trash cans, swimming and bathing pools/tubs, cat's litter boxes, animal beds, dog hygiene, pheromone pills, tampons, latex gloves, anti-mosquito candies, anti-mosquito skin cream, anti-mosquito agents, toothpaste, shower gel, body lotion, skin cream, hair gels, skin lotion, lipstick, fingernail/nail polish, skin whitening,
   b) applications and products in the food sector, e.g. chewing gum, tea, pills against bad breath, coffee, spices, frozen goods (pizza, etc.), pastries/baking goods, deodorant candies, drinks, taste enhancers, blocking of negative tastes, bitter taste inhibition, taste modulation,
   c) textiles, e.g. in detergent additives, softeners,
      Particularly preferred are applications in steam irons or ironers, even more preferred therein special applications in which the part cleaving thermally according to (1)b) is technically set exactly by choice of temperature and exposure time, and the not immediately released part of the odor in impregnated form stays as depot on the texiles/fabrics for future activation during use (particularly according to (1)a).
   d) applications and products in the construction sector, e.g. wood preservatives, wallpapers, building materials (mould control), odor color ("Geruchsfarbe"),
   e) applications in household products, e.g. scented candles, batteries, cigarettes, scented cards, 4D television, scent dispensers, candles, flowers.
(11) Product or composition containing one or more aroma glycosides in concentrations that are higher than their concentrations in natural products or compositions.
(12) Product or composition according to (11), wherein the aroma glycoside is produced biotechnologically.
(13) Product or composition according to (11) or (12), in which the concentration of the one or more aroma glycoside, respectively, is from 1-10000, preferably 10-1000, further preferably 20-200, most preferably around 100 ppm.
(14) Product or composition according to one of (11) - (13), which is a pharmaceutical or cosmetic product or composition, personal hygiene products, consumer articles, foods, nutraceuticals, food supplements, textile care products, household products or products in the construction sector.
(15) Glycosyltransferase, coded by a nucleic acid
   a) with the nucleotide sequence SEQ-ID1 or a nucleotide sequence which is at least 90%, preferably at least 95% or most preferably at least 98% identical to the SEQ-ID1, or a part thereof, which codes at least 50, preferably at least 80 or most preferably at least 200 amino acids,
   b) with a nucleotide sequence which is at least 90%, preferably at least 95% or most preferably at least 98% identical to the SEQ-ID2, or a part thereof, which codes at least 50, preferably at least 80 or most preferably at least 200 amino acids,
   c) with the nucleotide sequence SEQ-ID₃ or a nucleotide sequence which is at least 90%, preferably at least 95% or most preferably at least 98% identical to the SEQ-ID₃, or a part thereof, which codes at least 50, preferably at least 80 or most preferably at least 200 amino acids,
      (1') Product or composition, preferably fragrance article that can be activated, containing fragrance glycosides, wherein the fragrance glycosides are contained in concentration ranges that are higher than that of natural fragrance glycoside sources, and the fragrance article is used
         a) as fragrance article in the area of household products and promotional products, e.g. as scented candles, batteries, cigarettes, scented cards, 4D television, scent dispensers, candles, flowers, adhesive strips, strips, patch/band-aid.
         b) as insect repellent products, e.g. with candles and in scenting of rooms.
      (2') Product or composition according to (1'), wherein - beside the fragrance glycosides - an activating enzyme, e.g. β-glucosidase, is contained.
      (3') Product or composition according to (1') and/or (2'), wherein the fragrances can be activated according to need by
         a) biological mechanisms, e.g. an addition of enzymes, e.g. β-glucosidase,
         b) physical mechanisms, e.g. an increase of temperature, e.g. above 70°C,
         c) chemical mechanisms, e.g. a change in pH, e.g. below a pH of 3,
         d) according to (2) only through addition of water.
      (4') Product or composition according to (2'), wherein a repeated activation is possible by having a phase of drying after the first activation.
      (5') Product or composition according to (1'), in which
         a) the released aglycones are understood as e.g. odorous substances/scents, perfume oils, odors/smells, odorous substance compositions, odor compositions and the like.
         b) the released aglycones are for example geraniol, nerol, citronellol, thymol, carvacrol, eugenol, phenyl ethanol, vanillin, menthol, hexenol, anisole, butanethiol, decalactone, ethylguaiacol, farnesol, furfuryl alcohol, furfuryl mercaptan, guaiacol, heptyl alcohol, linalool, methenthiol, octalactone, propanethiol, raspberry ketone, terpineol, 10-undecenol, hydroxycitronellol, dihydromyrcenol, borneol, hesperetin, norfuraneol and ethyl maltol; particularly preferred are according to (3)a), b), c), d): furaneol, homofuraneol, caramel furanone, furanthiol, maltol, ethyl maltol, octenol, sotolon.
         c) the contained aglycones are organic alcohols that have been glycosylated, e.g. from the list of aroma agents approved in Europe (union list).
         d) the fragrance precursors are terpene glycosides, e.g. glycosylated terpenoids with one or more hydroxy functions (CHEBI:61777 list).
         e) the contained aglycones are glycosylated with a proportion, e.g. molar proportion, of one or a multitude thereof with glucose, arabinose, xylose, rhamnose, apiose or galactose.
      (6') Product or composition of (1'), wherein the fragrance glycosides are produced biotechnolgically.
      (7') Product or composition of (1'), in which the concentration of the one or more fragrance glycosides, respectively, is from 1-10000, preferably 10-1000, further preferably 20-200, most preferably around 100 ppm.
      (8') Product or composition of (1'), which is an artificial flower, for example without a stem as swimming flower, for example as flower with absorbing stem which transfers water from a glass to the flower / flower blosson and releases the fragrance there.
      (9') Product or composition of (8'), optionally with one or more properties of the following list:
         a) The flower petals additionally have a color that can be activated by water.
         b) The artificial stigma is formed as a depot in spherical (full sphere or coated hollow sphere) form to take up bigger amounts of fragrance that can be activated and of enzyme. Thereby, whole rooms can be scented.
         c) Using smart papers a spatial change can be achieved by water supply, and thereby e.g. the opening of a closed flower can be mimicked.
         d) Surprising artistic effects can be displayed by an intelligent arrangement of origami components.
      (10') Product or composition of (1'), which is a stripe, preferably self-adhering, preferably equipped with a depot, e.g. a fleece/non-woven, e.g. with a length of 1 cm to 100 cm without a restriction regarding width and height, for versatile scenting in wet rooms or usage with promotional items/articles.

### Examples

The present invention will now be described in detail with reference to several examples thereof. However, these examples are illustrative and do not limit the scope of the invention.

### Cosmetics / hygiene

### Example 1 (not according to the invention)

### Fragrances in cosmetic articles that can be activated

By adding aromas/aroma agents and fragrances to cosmetic articles the fragrant effect can be prolonged. For example the fragrant effect of a deodorant can be prolonged (see Fig. 1, 4GENE fragrance mixture). The activation of the fragrance precursors occurs over a prolonged period of time through the enzymes of the present skin bacteria (see Figure 4, dashed line). Below this threshold value no odor is perceptible anymore. The usual odor mixture (top note, solid line) has been volatilized after 3 hours, whereas the fragrance precursor mixture (4GENE fragrance mixture) is perceptible over 15 hours.

### Example 2 (not according to the invention)

### Wetness display through fragrances that can be activated in diapers

The fragrances that can be activated are put into a cellulose layer of a diaper in a dry state, through humidity and/or the bacterial enzymes contained in the excrements a release of the fragrance component is enabled, which serves as signal for the diaper change and at the same time covers bad smells.

### Example 3 (not according to the invention)

### Glycosides that can be activated for use in shoe soles

Shoe soles are treated with a spray which contains the fragrances that can be activated. The bacteria in the foot sweat activate the glycosides and prevent the unpleasant smell of the shoes during prolonged use in this way.

### Example 4 (not according to the invention)

### Glycosides that can be activated for use in textiles

The fragrances that can be activated can be added to the detergent or the softener so that they wet the textiles after washing. During wearing of the textiles the fragrance is only activated by sweat bacteria and thus protects the wearer of the textiles from bad smells.

Particularly innovative are applications in steam irons or ironers, in which the part cleaving thermally is technically set exactly by choice of temperature and exposure time (immediate scent effect), and the not immediately released part of the odor in impregnated form stays as depot on the texiles/fabrics for future activation during use.

### Foods

### Example 5 (not according to the invention)

### Food flavors that can be activated

Inactive aroma precursors are used in foods in order to release the aroma after activation. All foods are usable therefore which can be activated by temperature (> 70°C), microorganisms, addition of water and enzyme or influence of saliva. Examples thereof are foods which are heated during preparation like bread, cake and pizza, foods for which microorganisms are used for preparation, dry foods that come into contact with fluids like muesli and pulverized cold dishes, or foods that stay for a time in the mouth like chewing gum or drops/candies.

The proof-of-principle was now shown successfully for the example bread. Dough from three different kinds of bread was treated with the aroma precursor geranyl glucoside. In summary the breads with an addition of 100 ppm geranyl glucoside showed a conversion of about 6% glucoside to geraniol. This conversion is ascribed to the biggest extent to pyrolytic processes (about 5%) during the ten-minute baking process at 230°C and partly on the enzyme-catalyzed hydrolytic processes (about 1%) during the preparation of yeast- β-glucosidase.

The realization of a sensoric orthonasal test with testing the difference (triangle test) between bread with and without addition of geranyl glucoside for crust and crumb showed that the added amount of 100 ppm with a conversion of 6%, corresponding to a content of 1.7 mg/kg, respectively 1.7 ppm geraniol, in bread is sufficient to perceive a change of the total aroma.

The aromas that can be activated can be introduced into: semi-finished products, cake premixes, food supplements, sugar for burnt punch, waffles, crepes, hot fruit sauces, hot drinks, noodles. The release of the aromas from the precursors can be realized by: the cooking process, saliva, enzymes of microorganisms, baking, microwave, open fire, together with glycosidases only together with water.

### Baking test 1 - flower dough

Dough blanks of 10 g flour each were used as base. A negative control (flour, salt and water), three samples (added geranyl glucoside), a recovery (addition of geraniol to the bread prior to freezing according to a postulated conversion of glucoside of about 6% in order to determine the losses during the analytical process) and a control for geraniol in the glucoside solution (addition of an aliquot amount of glucoside only after the baking process to determine the content of geraniol). Several process steps are carried out according to standardized instructions, for each of the six blanks separately.

### Preparing the dough/paste:

For a blank/blind test (1) 9.98 g well-mixed flour were weighed into a glass vessel and 0.2 g NaCl were added. The mixture was at first mixed in a dry state for 1 min in a farinograph (= kneader) and kneaded for 8 min after pipetting 6.4 ml dist. water. The dough was taken out quickly and weighed for a first time (E1). A first fermenting period of 20 min at 31°C took place in a water vapor saturated proofing cabinet. After taking the dough out of the cabinet a second weighing (E2) took place as well as the homogenization. The ball-shaped blank was put into a round molder ("Rundwirker") and turned for 10 turns with a superimposed pestle. It was drawn through a pasta/noodle roller, folded two times without inclusion of air and again turned in the round molder for 20 turns. Finally a forming by hand was performed. After the homogenization a second fermentation period of 35 min follows.

For samples (2a-c) additionally 10 µl of a geranyl glucoside (GG) solution (c = 98 mg/ml) were quickly pipetted after the addition of water into the kneader.

In the control (3) no glucoside was added during preparation of the dough. The addition of 10 µl glucoside solution takes place for testing on impurities of the solution by geraniol immediately before reconditioning for the measurement to the baked, frozen bread.

In the recovery (4) no glucoside was added during preparation of the dough. The addition of 170 µl geraniol solution (1.24 mM) takes place for recovery of geraniol (0.0124 µmol/g bread, corresponding to a conversion of 6% GG) immediately before freezing with liquid nitrogen into the interior of the cooled bread.

### Baking

Baking took place on a tray in the oven for 10 min at 230°C. Before and after putting the samples vapor was put into the closed oven, respectively (spraying and evaporating of 25 ml dist. H₂O).

### Cooling/post-processing

The baked bread was left standing for 60 min at room temperature and subsequently frozen in liquid nitrogen. Storage of the breads took place at -18°C until the conditioning/preparation.

### Result

For the bread samples the following weights of Table 5 were recorded:

**Table 5: Mass of bread samples of baking test 1**

| **Sample** | **1** | **2a** | **2b** | **2C** | **3** | **4** |
|---|---|---|---|---|---|---|
| **E1 in g** | 15,9 | 15,5 | 15,7 | 15,9 | 15,7 | 16, |
| **E2 in g** | 15,9 | 15,5 | 15,7 | 16, | 15,7 | 16, |

After baking the breads were compared according to their appearance and smell in order to check first perceivable differences. All 5 breads showed a desired ball-like form of about same size. Inflation during the two-time fermentation only took place to a very small degree. Blank (negative) test (1.1), control (1.3) and recovery (1.4) showed a pale, hardly browned crust. The sample blanks (1.2a-c) had dark, brown spots. These came possibly from an increased content of glucose after release of the added glucoside and the increasingly taking-place Maillard-reaction resulting therefrom. Sample (2b) showed the coloration most pronounced, what indicates slightly changed conditions during baking.

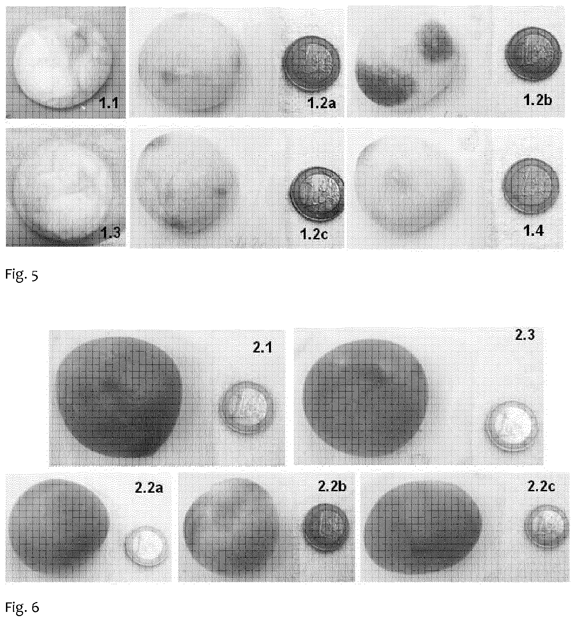

The results are shown in Fig. 5, pictures A1. From the smell samples 1, 3 and 4 reminded of the typically roasted, slightly sweet and intensive aroma of baked flour dough. With the sample blanks a slight fruity odor component could be perceived in addition after taking it from the oven, particularly at the bottom of the bread.

The sensory observations regarding the individual samples were in accordance to the expectations according to the different sample preparations with and without GG-dosage.

The analytical examination of the breads via GC resulted in the following measurement values of Table 6. For GC analysis, a Thermo Finnigan Trace DSQ mass spectrometer coupled to a Thermo Finnigan Trace GC was used. The instrument was equipped with a BPX5 20 M fused silica capillary column (30 m x 0.25 mm i.d., 0.25 µm film thickness). Helium gas flow rate was 1.1 ml/min. The injection port was operated in splitless mode. The injector temperature was 250°C, the ion source and interface temperatures were kept at 230 and 280 °C, respectively. The GC parameters were as follows: initial temperature 40 °C for 3 min, increased to 100 °C at a rate of 10 °C/min and from 100 °C to 190 °C at a rate of 30 °C/min. The EI-MS ionization voltage was 70 eV. Mass data was acquired in the range of 30-300 m/z. Compounds were identified by comparing their mass spectra and retention indices to the NIST mass spectra library and reference compounds.

The blind sample 1 as well as control 3 showed no geraniol sign/peak. The negative proof of control 3 (post-addition of GG stock solution after the baking process into the cooled bread) showed that no residue contents of geraniol due to impurities or decomposition processes were present in the GG stock solution and falsifications/errors could be excluded. The contents of geraniol in the bread samples therefore were determined directly from the peak areas of the extract measurements of the samples, with the results shown in Tables 7 and 8.

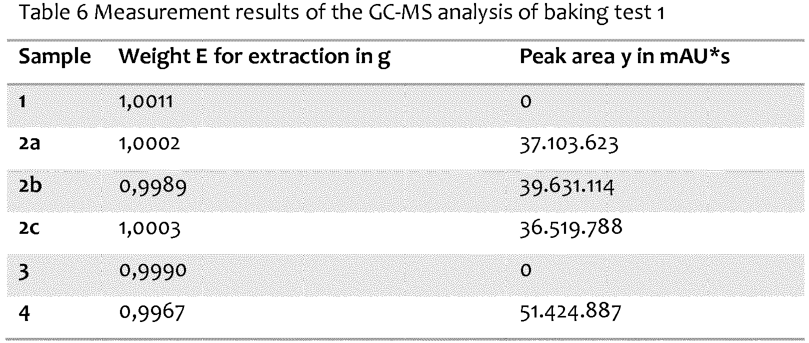

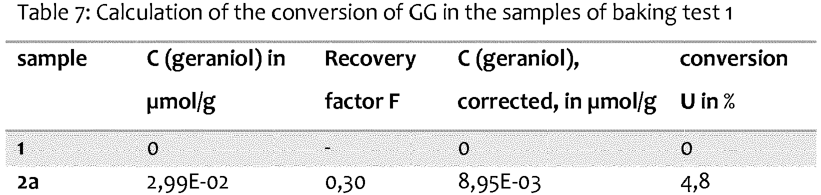

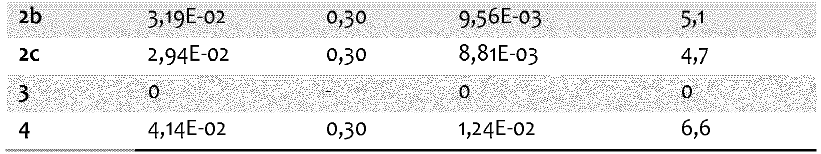

Averaging resulted in the following:

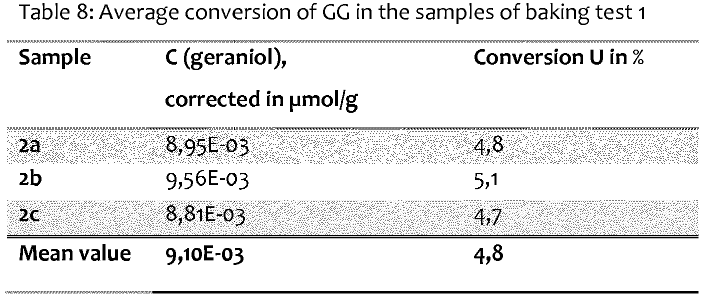

With a recovery factor of 0.30, calculated from adding geraniol to the recovery sample before freezing (4), quite stable measurement results were achieved for the respective samples with 4.7% (2c) to 5.1% (2b) conversion of GG to geraniol in the baking test with flour dough. The slightly deviating result of sample 2b is most likely related to the changed conditions during the bread preparation. It seems contradictory that the named sample has lower contents of geraniol in spite of a stronger browning. Since the difference could not be tackled exactly all three results were taken for averaging. The calculated mean value of the triplicate determination was therefore 4.8 % conversion from 3.2 µmol GG in 17 g bread based on flour dough. The cited conversion was already perceivable as fruity component of the crust aroma after baking.

### Baking test 2 - simple yeast dough

As samples dough blanks of 10 g flour each were used. A negative control (flour, salt, sugar, yeast and water), three samples (additionally geranyl glucoside) and a recovery (addition of geraniol to the bread before freezing according to a postulated conversion of glucoside of 6% in order to determine losses during the analytical procedure) were used. Several process steps were carried out according to standardized specifications, separate for each of the five blanks.

### Preparing the dough/paste:

For a blank/blind test (1) 9.98 g well-mixed flour were weighed into a glass vessel. 0.2 g NaCl and 0.1 g sucrose were added and the mixture was put in the kneader. An addition of 0.7 g (cooled) baking yeast directly to the kneader followed. The mixture was at first kneaded in a dry state for 1 min and kneaded for 8 min after pipetting 6.3 ml dist. H₂O. All further steps including the work-up and sample taking ware carried out according to the sample with flour dough (1). In the samples (2a-c) 10 µl GG-solution were pipetted directly after the addition of water to the kneader. The preparation of the recovery (3) was carried out analogous to the blind sample. After cooling for one hour 170 µl geraniol solution were injected herein into the breadcrumb directly before freezing with liquid nitrogen.

### Result

For the bread samples the following weights of Table 9 were recorded:

**Table 9: Mass of bread samples of baking test 2**

| **Sample** | **1** | **2a** | **2b** | **2C** | **3** |
|---|---|---|---|---|---|
| **E1 in g** | 16,3 | 16,7 | 16, | 16, | 16,94 |
| **E2 in g** | 16,3 | 16,5 | 16, | 16,7 | 16,68 |

All five breads showed a bigger volume and stronger coloring of the crust compared to the baking test based on flour dough. The inflating during the fermentation process is attributed to the formation of gases due to the added yeast, which are held back due to the net-like dough structure. The strong brown color resulted either from an increased glucose and protein content or stronger heating processes in the crust, which are related to the changed bread structure. Striking was the irregular browning of sample 2b in contrast to the two other bread samples. The reason for this is an erroneous production of the bread. Contrary to the specification two homogenization steps were carried out during preparation of the dough, which lead to a stronger densification of the bread blank. As a result also the degree of the release of geraniol is influenced to an unknown degree due to the changed thermo and pyrolysis conditions. This circumstance had to be taken into account during evaluation of the baking test.

The results are shown in Fig. 6, pictures A2. The olfactory examination of blind sample (1) and recovery (3) showed in this experiment a typical yeast-sour, roasty, intensive baking aroma which showed the addition of yeast clearly. As already observed in baking test 1 a fruity note could be perceived in the total aroma in the samples with added GG solution (2a-c) directly after taking out of the oven, which thus reminded strongly of the scent of fine baking products (croissant).

The GC-MS analysis showed the following results of Table 10.

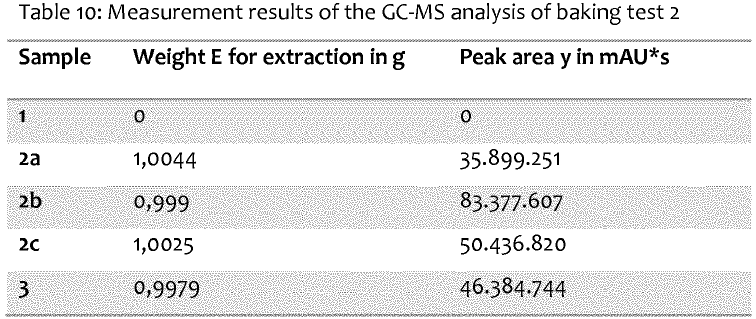

The measurement of blind sample1 showed no geraniol peak. The amounts of geraniol in the bread samples are shown in Table 11.

A recovery factor of 0.33 was found from the quantification of the recovery sample. In the samples of this baking test the strong variation between the measurement results of the three breads was apparent, whose conversion was between 5.1% (2a) and 11.8% (2b). Since the higher result for sample 2b could be attributed to the deviations during the preparation of the bread, only samples 2a and 2c were taken into account during averaging of the results, the results of which are shown in Table 12.

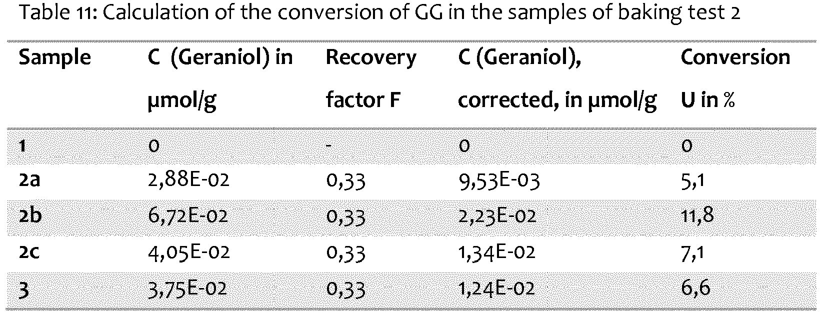

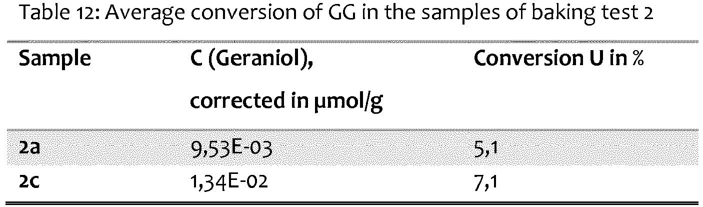

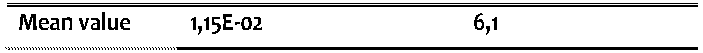

The calculated mean value of the two-fold determination resulted in a conversion of 6.1% from 3.2 µmol GG in 17 g bread on the basis of a simple yeast dough. The obtained results were 1.2% higher than the one of baking test 1 with bread from flour dough (4.9%).

### Baking test 3 - standard yeast dough

As samples dough blanks of 10 g flour each were used. A negative control (flour, salt, sugar, fat, yeast, L-ascorbic acid and water), three samples (additionally geranyl glucoside) and a recovery (addition of geraniol to the bread before freezing according to a postulated conversion of glucoside of 6% in order to determine losses during the analytical procedure) were used. Several process steps were carried out according to standardized specifications, separate for each of the five blanks.

### Preparing the dough/paste:

For a blank/blind test (1) 9.98 g well-mixed flour were weighed into a glass vessel. 0.2 g NaCl and 0.1 g sucrose and 0.1 g palm fat were added and the mixture was put in the kneader. An addition of 0.7 g (cooled) baking yeast directly to the kneader followed. The mixture was at first kneaded in a dry state for 1 min and kneaded for 8 min after pipetting 6.0 ml dist. H₂O and 0.3 ml ascorbic acid solution.

All further steps including the work-up and sample taking ware carried out according to the sample with flour dough (1). In the samples (2a-c) 10 µl GG-solution were pipetted directly after the addition of water and ascorbic acid solution to the kneader. The preparation of the recovery (3) was carried out analogous to the blind sample. After cooling for one hour 170 µl geraniol solution were injected herein into the breadcrumb directly before freezing with liquid nitrogen.

### Result

For the bread samples the following weights of Table 13 were recorded:

**Table 13: Mass of bread samples of baking test 3**

| **Sample** | **1** | **2a** | **2b** | **2c** | **3** |
|---|---|---|---|---|---|
| **E1 in g** | 17,0 | 16, | 16, | 16, | 16.90 |
| **E2 in g** | 16, | 16, | 16, | 16, | 16,87 |

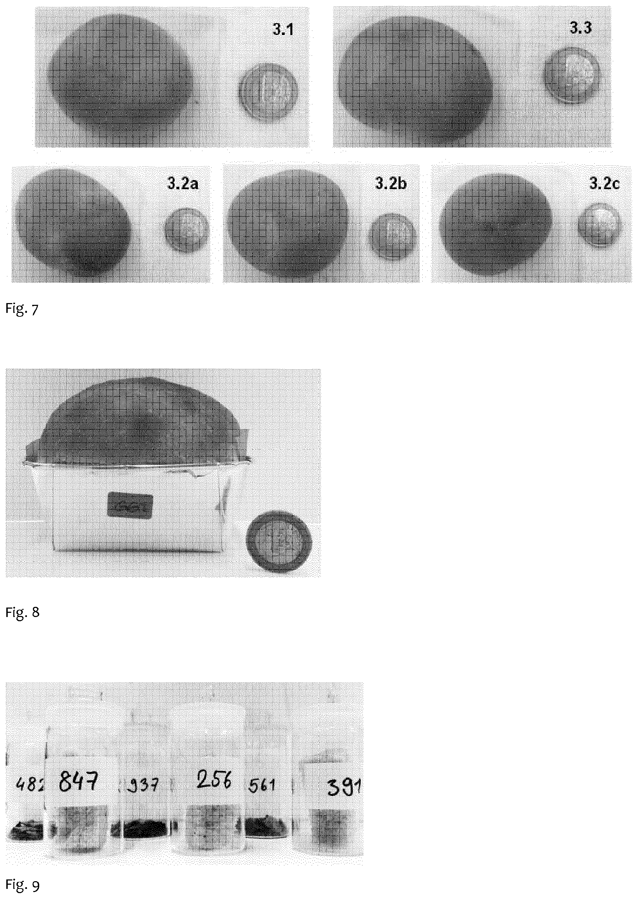

All five breads showed a similarly increased volume after baking as in the baking test with simple yeast dough. The breads were all colored brown uniformly and strongly, so that no difference could be seen between the samples with and without precursor. The results are shown in Fig. 7, pictures A3. From an olfactory point the blind sample (1) and recovery (3) could not be differentiated from the analogue samples from baking test 2 with their typical yeast-like aroma. The samples (2a-c) showed a fatty fruity odor quality, as described before, which thus imparted the impression of fine baking products. In the aroma the respective breads of baking tests 2 and 3 are thus in accordance. The analysis of the release of geraniol in these experiments gave the following results shown in Table 14.

The measurement of blind sample 1 again showed no geraniol peak.

From the calculation the following values of Table 15 are obtained.

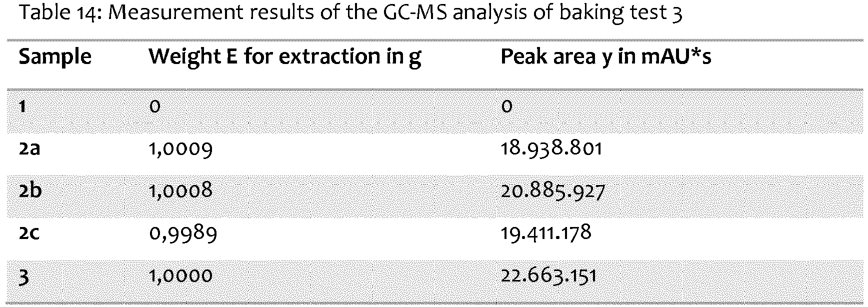

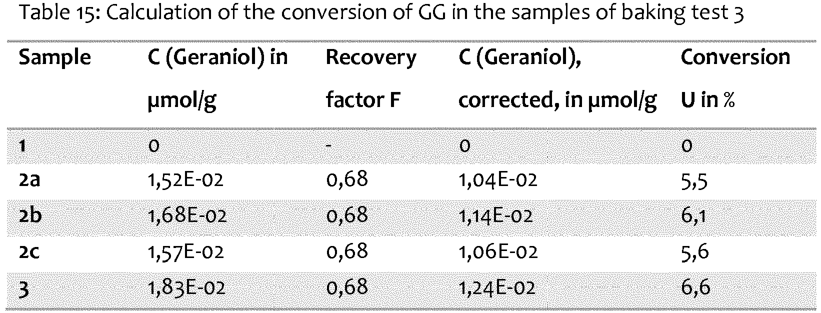

A recovery factor of 0.68 was obtained from the quantification of the recovery sample. The samples showed sufficiently constant measurement values between 5.5% (2a) and 6.1% (2b) of GG in this experiment.

The averaging of the triple determination resulted in the values given in Table 16.

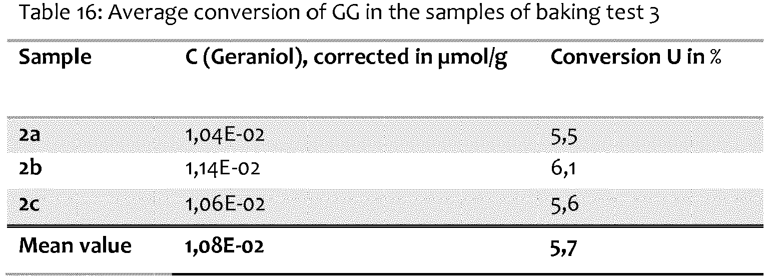

The calculated mean value of the triplicate determination resulted in a conversion of 5.7% from 3.2 µmol GG in 17 g bread based on standard yeast dough. It was thus a little under the value of conversion determined in baking test 2 (simple yeast dough without ascorbic acid) of 6.1% and above the one of baking test 1 (flour dough) of 4.8%, with the results summarized in Table 17.

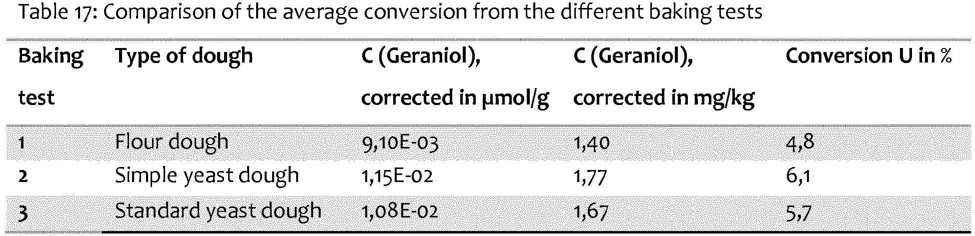

In total similarly low conversion rates of geranyl glucoside for the differently prepared baking tests were obtained. The bread samples based on yeast dough showed similarly pronounced results with 6.1% (simple yeast dough) and 5.7% conversion (standard yeast dough). In the bread this translated to a content of geraniol of 1.77 and 1.67 mg/kg, respectively. Bread based on flour dough showed a result about 1% lower with a conversion of 4.8% GG, what corresponds to a content of 1.40 mg/kg geraniol in the baked bread.

A statement if the determined conversion of geranyl glucoside to aromatic geraniol can also be perceived by untrained consumer resulted in the following sensory test.

### Sensory test

In order to test the influence of the amount of geraniol released in the baking test on the total aroma of the breads and its impression on the consumer an orthonasalic sensory test was carried out. In a triangle test with untrained panelists breads based on standard yeast dough with and without addition of GG were tested for a perceivable difference.

Bread made of standard yeast dough was tested analogous to the amount of 100 ppm geraniol (corresponds to 1 mg/10 g) used until now in raw bread dough and its difference to a blind sample. As sample dough blanks of 50 g flour each based on standard yeast dough (baking test 3 - standard yeast dough) were used (see Fig. 8, picture A4) A blind sample A (flour, salt, sugar, fat, yeast, L-ascorbic acid and water) and a positive control B (additionally geranyl glucoside) were used. Several process steps were carried out according to standardized specifications, separate for each of the two blanks.

### Preparing the dough/paste:

For the blind sample A 94.9 g well-mixed flour were weighed into a glass vessel. 1.0 g NaCl, 0.5 g sucrose and 0.5 g palm fat were added and the mixture was put in the kneader. An addition of 3.5 g (cooled) baking yeast directly to the kneader followed. The mixture was at first kneaded in a dry state for 1 min and kneaded for 8 min after addition of 30.0 ml dist. H₂O and 1.5 ml ascorbic acid solution. For the positive sample B additionally 50 µl of a GG stock solution (c = 98 mg/ml) were pipetted quickly after the addition of water and ascorbic acid to the kneader.

The dough was taken out quickly and weighed a first time (E1). A first fermenting period of 20 min at 31°C took place in a water vapor saturated proofing cabinet. After taking the dough out of the cabinet a homogenization took place. The ball-shaped blank was put into a round molder and turned for 10 turns. It was rolled flat to a height of 1 cm, folded two times without inclusion of air and again turned in the round molder for 20 turns. Finally a forming by hand to form a roll was performed. This roll was put into a small loaf pan made of aluminum and weighed again (E2). After the homogenization a second fermentation period of 40 min followed.

### Baking

Baking took place in the oven for 20 min at 230°C. Before and after putting the blank vapor was put into the closed oven, respectively (spraying and evaporating of 25 ml dist. H₂O).

### Cooling/preparation

The baked bread was left standing for 60 min at room temperature and subsequently prepared for the sensory test. For testing the crust it was taken off with a knife, simultaneously shredded and 1g each weighed into an airtight sensory vessel made of glass with ground joint and cover/lid. For preparing the crumb the bread that was totally freed from the crust was at first cut into 1.5 cm thick slices using a bread slicer and separated with an electronic knife into cuboids with a size of about 1.5 x 5.0 x 2.0 cm³ and a weight of 4.0 g. A cuboid each was put into an airtight sensory vessel made of glass with ground joint and cover/lid. Directly thereafter the sensory test took place.

### Test

The process of the sensory test followed a simple triangle test with a test for differences based on DIN/ISO 4120 (Busch-Stockfisch, 2008). Bread crust and bread crumb of two charges of bread A and B were respectively tested in a triad of three filled sensory vessels from 18 panelists each orthonasal for a perceptible difference, wherein two vessels contained identical samples and the third was filled differently. All three samples were encoded with three-digit number codes. The following sample assemblies resulted:
Crust:

| B | B | A |
|---|---|---|
| (937) | (482) | (561) |

and

| A | A | B |
|---|---|---|
| (561) | (208) | (482) |

Crumb:

| A | B | A |
|---|---|---|
| (391) | (256) | (847) |

and

| B | A | B |
|---|---|---|
| (256) | (391) | (408) |

Each panelist carried out two tests following each other, first for the sensory test of the crust, then for the crumb. After-tasting of the vessels within a test was allowed, a decision was forced (Forced-Choice-Principle). Following the test all singular results were brought together and evaluated statistically.

### Result

In a basic protocol the results of the single protocols were brought together and evaluated statistically to test for a significance of the difference.

For this purpose the extent of the α-risk/ α-error was determined based on the relation of correct and wrong answers: the probability of the conclusion that a perceivable difference is present although this is not the case (also termed error of the first order/type, significance lever or probability of an erroneous rejection).

The α-risk was determined using suitable significance tables.

Valid is the following:

| | | |
|---|---|---|
| α ≤ 0,05 (5%) | n. s. | = not significant α = < 0,05 (5 %) |
| α = 0,05 (5%) | s | = significant |
| α = 0,01 (1 %) | h. s. | = highly significant |
| α = 0,001 (0,1%) | s. h. s. | = very highly significant |

The test showed the following result of Table 18.

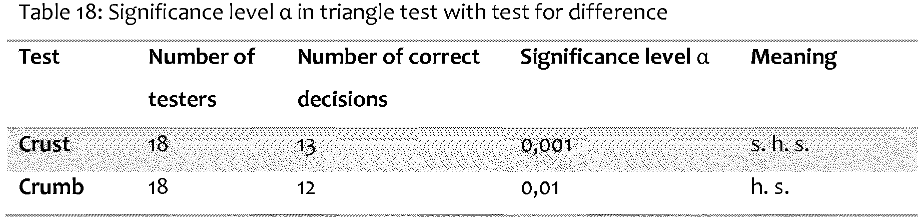

The evaluation of both tests showed that most of the panelists could perceive a difference between the blind sample and bread. When checking the significance level an α -value of 1% resulted in the sensory test of the crumb with 12 correct answers for 18 panelists. This confirmed that the breads actually could be differentiated according to their smell and no difference that was not present was erroneously perceived. In the test for difference of the aroma of the crust an α -value of 0.1% was achieved with 13 correct answers for also 18 panelists, and thus a highly significant result (see Fig. 9, picture A5).

The application of geranyl glucoside as additive of bread in the described way and an appropriate dosing is thus suitable for producing an aromatized food. It could not be determined with the test if the fruity odor quality would be accepted by the consumer and, if yes, in which amounts. The results of the preference test clarified this.

### Taste modulation

The glycosylated aroma agents also have taste-modulating properties. The glycosylation can reduce or prevent a bitter taste or function as taste enhancer. In addition, the enhanced stability of the glycosides is also advantageous in the application as taste modulators.

### Example 6 (not according to the invention)

The experiments of Example 5 were repeated using furaneol instead of geraniol with amounts of the aroma at a factor 2 to 5 increased. The results of Example 5 could be confirmed with these experiments.

### Repellent

### Example 7 (not according to the invention)

### Glycosides that can be activated for application as repellent with long-term effect

A skin cream with e.g. citronellol glucoside can be e.g. used as repellent for mosquitoes (subfamilies Anophelinae and Culicinae). Therein the free citronellol is released by enzymes of skin bacteria from the citronellol glucoside over a prolonged period of time and enables a long-lasting mosquito protection. A similar effect can be achieved by taking up the citronellol glucoside in a candy orally and releasing the citronellol during digestion, which then is released in the whole body. This application is e.g. to be applied preferably during stays in the tropics to effectively repel mosquitoes that transfer sicknesses.

### Construction materials

### Example 8 (not according to the invention)

### Glycosides that can be activated for use in controlling mold

Construction materials, e.g. modern stones for building walls or insulation mats, etc., are added with the glycosides. Due to the fungicidal effect which only is activated enzymatically the mold can be controlled effectively. Only when mold wants to form and thus releases enzymes itself it kills itself this way.

### Electronics

### Example 9 (not according to the invention)

### Glycosides that can be activated for odor TV

The fragrances that can be activated can be stored in an inactive state for a very long time in contrast to the free fragrances. This predestines them for the application in odor TV (4D TV), wherein they can then be selectively released by electric heating.

### Example 10 (not according to the invention)

### Glycosides used for an electronic sensor system

The fragrances that can be activated can be used for an electronic sensor system. After activation by a trigger such as enzymes, temperature, or pH the volatile flavors / odors or the released sugars of the glycosides act as a signal for an electronic sensor system and can be measured with an electronic sensor.

### Household products

### Example 11 (not according to the invention)

### Artificial flowers and plants

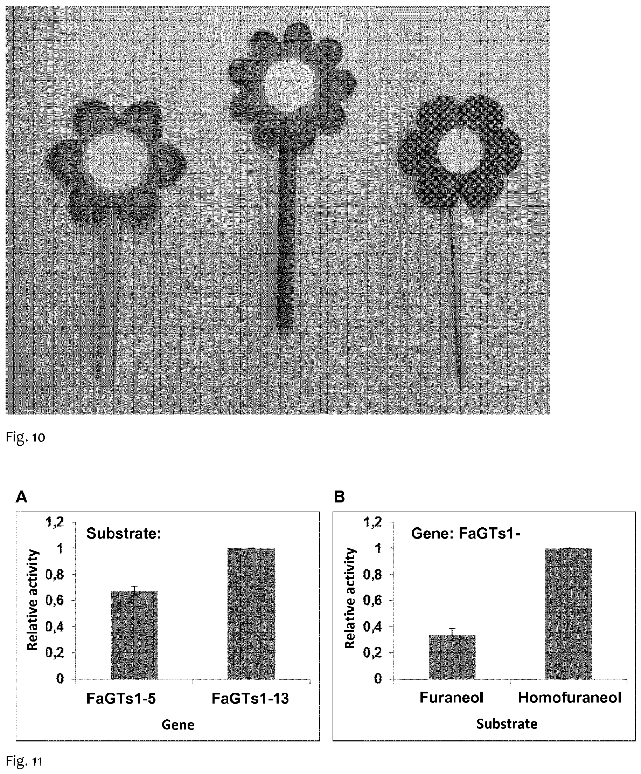

One further example is the use of inactivated fragrance precursors in an artificial flower. This consists of decorative artificial flower petals which surround a surface, e.g. a circle, or ball consisting of an absorbent material functioning as depot (see Fig. 10). This depot contains a dry mixture of a fragrance precursor, a glycoside, and a glycosidase, e.g. a glucosidase, in sufficient quantity. This artificial flower head is on top of a stem/stalk of water-conducting material, which, in turn can contain decorative leaves. This entity together forms an artificial flower.

When this artificial flower is put into a water container, e.g. a vase, water is lead through the absorbent material of the stem to the depot, in which the water enables the glycosidase, e.g. glucosidase to cleave the sugar of the fragrance precursor. The fragrance molecule thus formed is volatile and thus gets into the air, so that the artificial flower smells.

An experimental setup (proof-of-principle) was carried out as follows:
Solutions of different amounts of geranyl glucoside in ethanol were put on carrier materials with each a size of 2 cm², respectively, and dried. Afterwards 5 to 10 mg of glucosidase were added and pasted over with a carrier material. In a setup the amounts described in Table 19 were used.

**Table 19: Experimental setup of example 11**

| Glucosidase | Geranylglucosid | Water | Perceptible odor |
|---|---|---|---|
| 5mg | 2mg | 1 ml | weak |
| 10 mg | 5 mg | 1 ml | significant |
| 10 mg | 10 mg | 1 ml | significant |
| 10 mg | - | 1 ml | - |
| - | 2 mg | 1 ml | - |
| 10 mg | 5 mg | - | - |

The loaded carrier materials were then wetted with 1 mL water. With a usage of 5 mg geranyl glucoside a distinctly perceptible odor could be smelled. With 2 mg, a weak odor could be achieved, and without either glucosidase or geranyl glucoside or water no odor could be noticed, as expected. A follow-up experiment was to rewet already used carrier materials after drying again. Also here again a volatile odor was perceived. The developed aromatic flowers thus can be used repeatedly and are not disposable articles.

In a further example an artificial flower was recreated, which can be seen in Fig. 10. Again the proof-of-principle was repeated, but this time with an absorbent stem that transports water to the carrier material after the flower is put into a glass of water. The results of the first experiment were confirmed therewith.

Further derivatives are:
- The flower petals have colors that can be activated and that only become colored by water.
- The depot should be ball-shaped (full sphere or coated semi-sphere) so that bigger amounts of the fragrance that can be activated and the enzyme can be included. This way whole room can be scented.
- Using smart paper a spatial change can be accomplished through feeding water and e.g. the opening of a closed flower can be mimicked.
- Also origami-components are possible.
- Floating water lilies would not need the stem.
- Glycoside mixtures should be used so that the resulting fragrance can be smelled by as many people as possible.

In addition, the principle of artificial flowers can be transferred to other articles, e.g. scented balls which only release the fragrance after introduction into water, coated colored waterfalls for "fountain fragrance fireworks" ("Brunnenduftfeuerwerke"), self-adhering stripes that are furnished with an absorbent layer and can be adhered everywhere as fragrance dispensers after wetting, e.g. on promotional items.

### Further applications

This list of examples is not exhaustive. From Table 4 further applications can be derived. The basis is always the activation with subsequent effect, like e.g. aromatic, smelling, fungicidal, bactericidal or repelling for mosquitoes.

### New glycosyltransferases and glycosides

### Example 12

### Biotechnological production of furanon glycosides using glycosyltransferases

Glycosylated natural substances are important components of foods, pharmaceutical and cosmetic products due to their manifold physiological activities. For example, glycosylated diterpenes, steroids and flavonoids are used as sweeteners (steviosides, glycyrrhizin, neohesperidin) in the food industry, while steroid and antibiotic glycosides are used as medicines and glycoside extracts as cosmetic agents. Chemical-synthetic glycosylation reactions of the take place in the presence of heavy-metal containing catalysts, so that therefore recently biocatalysts are sought which can catalyze this reaction alternatively. Products obtained via biocatalysts correspond to the natural substances, are free of heavy metal residues and can therefore be declared as "natural" according to present law, which is a direct advantage for the consumer and for industry an indirect advantage in marketing. In nature, regio- and enantioselective sugar transfers are catalyzed by nucleoside diphosphate carbohydrate (e.g. UDP glucose) depending glycosyltransferases (UGTs). Glycosyltransferases that convert low molecular substrates transfer sugar on a plurality of acceptors like hormones and secondary metabolites as well as biotic and abiotic chemicals and toxins. The enzymes are coded in the different organisms by a big multigene family and can be identified by a common motif in their primary sequence (PSPG Box, Plant Secondary Product Glycosyltransferase). The transfer of a carbohydrate residue to a mostly lipophilic acceptor changes its chemical properties and its bioactivity and can facilitate the access to cellular membrane transporter systems. In vitro studies show that a singular UGT protein can glycosylate several substrates of different origin, but also that several UGTs can glycosylate the same substrate, which makes the identification of substrate specificity difficult to predict and thus impedes it.

Due to the sequencing of genomes of different organisms on a huge scale now continually many new UGT sequences are being published. During sampling of the UGT gene family of plant species which are particularly known for the production of glycosides sequences could be isolated now which code for UGT enzymes which catalyze the glycosylation of aromas / aroma agents very efficiently.

### Own technical solution

In the search for efficient biocatalysts which can be particularly used for the production of aroma glycosides the UGT gene family of plant species *Vitis vinifera* (grape vine) and *Fragaria x ananassa* (strawberry) was examined. For this at first the gene sequences were searched that are strongly expressed in the fruits of the plant species. These were subsequently isolated, expressed in the host organism *Escherichia coli,* and the enzymatic activity toward different potential substrates was determined. At least three of the coding nucleic acid sequences (FaGTs1-5, FaGTs1-13 und VvdGT13, see appended sequences) catalyze the formation of aroma glycosides very efficiently.

### The essential new

During the transfer of the plant gene sequence to *E.coli* unexpectedly an additional sequence was obtained. The corresponding protein sequence shows at positions 222 and 323 in place of histidine and aspartic acid the amino acids tyrosine and glutamic acid. This sequence was termed by us FaGTs1-13 and included in further experiments. A result thereof was that this new sequence has a 30% increased activity - in relation to molar conversion - compared to sequence FaGTs1-5 (see Fig. 11 A).

In Fig. 11 the determination of the enzyme activity of FaGTs1-5 and FaGTs1-13 is shown. Fig. 11A shows the relative activities of FaGTs1-5 and FaGTs1-13 for homofuraneol, and Fig. 11B shows the relative activities of FaGTs1-13 with different substrates, i.e. furnaeol and homofuraneol, wherein the highest values were normalized to 1.

Unexpectedly, the biocatalysts FaGTs1-5 and FaGTs1-13 produced the glucosides of homofuraneol (Fig. 11B) and norfuraneol apart from the glucosides of furaneol, both of which are not present in the examined plant species. The homofuraneol-β-D-glucoside, which for the first time can be produced using the biocatalysts FaGTs1-5 and FaGTs1-13, was not described previously. Surprisingly homofuraneol is converted 3 times more effectively than furaneol (Fig. 11B).

The components for the enzymatic reaction are shown in Table 20.

**Table 20: Pipetting scheme for the in vitro assay**

| | |
|---|---|
| Tris buffer (0.,1 M, pH 7.5) | 100 µl |
| Substrate (60 mM) | 2 µl |
| UDP-glucose (¹²C) 0.2 mM | 49.9 µl |
| UDP-glucose (¹⁴C) 0.3 mM | 0.1 µl |
| Enzyme | 2 µg |

→Ad 200 µl with H2O
→1 h at 30 °C incubation

The reaction was started by adding UDP-glucose and stopped after incubation with 1 ml n-butanol. After vortexing for at least 15 s the reaction mixture was centrifuged 2 min at 12 000 xg for separation of the phases, and 800 µl of the upper organic phase containing the product were now transferred to 2 ml scintillation cocktail (Ultima-Flo AF, PerkinElmer). Non-converted UDP glucose remains in the aqueous phase and thus does not contribute to the radioactivity intensity in the sample (organic phase).

In whole cell fermentations wherein "resting cells" were incubated in M9 minimal medium (22 mM KH2PO4, 47.8 mM Na2HP04.2H2O, 19 mM NH4Cl, 8.6 mM NaCl, 0.1 mM CaCl2, 2 mM MgSO4, 1 % glucose, pH 7.0) with the furanones more than 1 g/l furanon glycosides could be biotechnologically produced without any optimization until now. The simple production of biocatalysts in big amounts, the *in vivo* production of the co-substrate UDP-glucose in the cells and the abundant product amounts facilitate now the possibility to produce the target substances in an economical scale for industrial applications.

The products described herein enable for the first time the appropriate, biologically, physically and pH chemically controlled activation of flavorings and fragrances instead of the physical-chemical release of flavorings and fragrances used until now.

The glycosyltransferase method described herein is highly innovative. It is based on a natural principle, uses modern methods of biotechnology and is already very efficient on a laboratory scale. It is possible to produce the required amounts of flavoring and fragrance glycosides with it. It is unparalleled and in an unique position.

## Claims

1. Glycosyltransferase for producing an aroma glycoside coded by a nucleic acid sequence that
a) comprises the sequence of SEQ-ID2.

2. The glycosyltransferase according to claim 1, wherein the glycosyltransferase is capable of catalyzing formation of a glycoside in which a sugar is linked to an aglycone through a β-D-glycosyl linkage.

3. A nucleic acid molecule encoding a glycosyltransferase as defined in claim 1 or 2, wherein said nucleic acid molecule is a DNA molecule, particularly a cDNA molecule.

4. A vector comprising a DNA sequence encoding a glycosyltransferase as defined in claim 1 or 2.

5. A host cell containing or transfected with the heterologous nucleic acid molecule according to claim 3 or the vector according to claim 4.

6. A transgenic plant comprising a heterologous nucleic acid molecule as defined in claim 3 or a vector as defined in claim 4.

7. A method of forming at least one aroma glycoside, wherein an aglycone is covalently linked to a sugar donor comprising contacting the aglycone with the sugar donor and a glycosyltransferase as defined in any of claims 1 or 2 under conditions appropriate for the transfer of the sugar group of said sugar donor.

8. The method of claim 7, comprising the steps of:
- culturing or growing a host cell as defined in claim 5 or a transgenic plant as defined in claim 6; and
- collecting from said host cell or transgenic plant said aroma glycoside.

## Patentansprüche

1. Glykosyltransferase zur Herstellung eines Aroma-Glykosids, kodiert durch eine Nukleinsäuresequenz, die
a) die Sequenz von SEQ-ID2 enthält.

2. Glykosyltransferase nach Anspruch 1, wobei die Glykosyltransferase imstande ist, die Bildung eines Glykosids zu katalysieren, in dem ein Zucker an ein Aglykon durch eine β-D-Glykosyl-Bindung gebunden ist.

3. Nukleinsäuremolekül, das eine Glykosyltransferase wie in Anspruch 1 oder 2 definiert kodiert, wobei das Nukleinsäuremolekül ein DNA-Molekül, vorzugsweise ein cDNA-Molekül, ist.

4. Vektor, umfassend eine DNA-Sequenz, die eine Glykosyltransferase wie in Anspruch 1 oder 2 definiert kodiert.

5. Wirtszelle, die das heterologe Nukleinsäuremolekül nach Anspruch 3 oder der Vektor nach Anspruch 4 enthält oder mit ihm transfiziert ist.

6. Transgene Pflanze, umfassend ein heterologes Nukleinsäuremolekül wie in Anspruch 3 definiert oder einen Vektor wie in Anspruch 4 definiert.

7. Verfahren zum Bilden von mindestens einem Aroma-Glykosid, wobei ein Aglykon kovalent an einen Zucker-Donor gebunden ist, umfassend ein Kontaktieren des Aglykons mit dem Zucker-Donor und einer Glykosyltransferase wie in einem der Ansprüche 1 oder 2 definiert unter Bedingungen, die für den Transfer der Zuckergruppe des Zucker-Donors geeignet sind.

8. Verfahren nach Anspruch 7, umfassend die Schritte:
- Kultivieren oder Züchten einer Wirtszelle wie in Anspruch 5 definiert, oder einer transgenen Pflanze wie in Anspruch 6 definiert; und
- Sammeln des Aroma-Glykosids aus der Wirtszelle oder der transgenen Pflanze.

## Revendications

1. Glycosyltransférase pour produire un glycoside aromatique codée par une séquence d'acide nucléique qui
a) comprend la séquence de SEQ ID2.

2. Glycosyltransférase selon la revendication 1, dans laquelle la glycosyltransférase est capable de catalyser la formation d'un glycoside dans lequel un sucre est lié à un aglycone par une liaison β-D-glycosyle.

3. Molécule d'acide nucléique codant pour une glycosyltrasférase telle que définie dans la revendication 1 ou 2, dans laquelle ladite molécule d'acide nucléique est une molécule d'ADN, en particulier une molécule d'ADNc.

4. Vecteur comprenant une séquence d'ADN codant pour une glycosyltransfrase telle que définie dans la revendication 1 ou 2.

5. Cellule hôte contenant ou transfectée avec la molécule d'acide nucléique hétérologue selon la revendication 3 ou le vecteur selon la revendication 4.

6. Plante transgénique comprenant une molécule d'ADN hétérologue telle que définie dans la revendication 3 ou un vecteur tel que défini dans la revendication 4.

7. Procédé de formation d'au moins un glycoside aromatique, dans lequel un aglycone est lié de manière covalente à un donneur de sucre comprenant la mise en contact de l'aglycone avec le donneur de sucre et une glycosyltransférase telle que définie dans l'une quelconque des revendications 1 ou 2 dans des conditions appropriées pour le transfert du groupe de sucre dudit donneur de sucre.

8. Procédé selon la revendication 7, comprenant les étapes de :
- culture ou croissance d'une cellule hôte telle que définie dans la revendication 5 ou une plante transgénique telle que définie dans la revendication 6 ; et
- collecte de ladite cellule hôte ou de la plante transgénique dudit glycoside aromatique.
